# EUROPÄISCHE PATENTSCHRIFT

(11) **EP 1 853 608 B1**
(45) Veröffentlichungstag und Bekanntmachung des Hinweises auf die Patenterteilung: **09.07.2008**
(21) Anmeldenummer: 06708259.4
(22) Anmeldetag: 14.02.2006
(51) Int. Cl.: C07D 487/04, A01N 43/90

(54) **5-ALKOXYALKYL-6-ALKYL-7-AMINO-AZOLOPYRIMIDINE, VERFAHREN ZU IHRER HERSTELLUNG UND IHRE VERWENDUNG ZUR BEKÄMPFUNG VON SCHADPILZEN SOWIE SIE ENTHALTENDE MITTEL**
5-ALKOXYALKYL-6-ALKYL-7-AMINO-AZOLOPYRIMIDINES, METHOD FOR THEIR PRODUCTION, THEIR USE FOR CONTROLLING PATHOGENIC FUNGI AND AGENTS CONTAINING SAID SUBSTANCES
5-ALKOXYALKYL-6-ALKYL-7-AMINO-AZOLOPYRIMIDINES, PROCEDE DE FABRICATION DE CES COMPOSES, UTILISATION DANS LA LUTTE CONTRE DES CHAMPIGNONS PARASITES ET AGENTS LES CONTENANT

(30) Priorität: 16.02.2005 DE 102005007157
(43) Veröffentlichungstag der Anmeldung: 14.11.2007
(73) Patentinhaber: BASF SE, 67056 Ludwigshafen (DE)
(72) Erfinder: SCHÄFER, Peter, 67308 Ottersheim (DE); HÜNGER, Udo, 68167 Mannheim (DE); SCHERER, Maria, 76829 Landau (DE); KÖHLE, Harald, 67273 Bobenheim (DE); SCHIFFER, Helmut, 67483 Grossfischlingen (DE); GROTE, Thomas, 67157 Wachenheim (DE); DIETZ, Jochen, 68167 Mannheim (DE); GRAMMENOS, Wassilios, 67071 Ludwigshafen (DE); LOHMANN, Jan, Klaas, 68167 Mannheim (DE); MÜLLER, Bernd, 67227 Frankenthal (DE); RHEINHEIMER, Joachim, 67063 Ludwigshafen (DE); SCHIEWECK, Frank, 67098 Bad Dürkheim (DE); SCHWÖGLER, Anja, 68165 Mannheim (DE)
(86) Internationale Anmeldenummer: PCT/EP2006/050922
(87) Internationale Veröffentlichungsnummer: WO 2006/087325

(56) Entgegenhaltungen:
- EP-A- 0 141 317
- EP-A2- 0 217 218
- WO-A1-98/33799
- DE-A1- 1 620 694

## Beschreibung

Die vorliegende Erfindung betrifft 5-Alkoxyalkyl-6-alkyl-7-amino-azolopyrimidine der Formel I 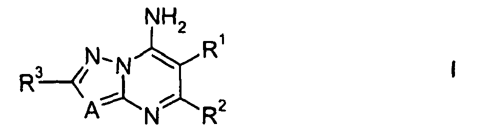 in der die Substituenten folgende Bedeutung haben:
- R¹: C₁-C₁₂-Alkyl, C₃-C₆-Cycloalkyl, C₂-C₁₂-Alkenyl, C₂-C₁₂-Alkinyl, C₂-C₁₂-Alkoxyalkyl, C₂-C₁₂-Cyanoalkyl und C₈-C₁₉-Benzyloxyalkyl, wobei die Gruppen im aliphatischen oder aromatischen Teil unsubstituiert oder durch eine bis drei Gruppen R^{a} substituiert sein können:
R^{a} Halogen, Cyano, Nitro, Hydroxy, C₃-C₆-Cycloalkyl, C₁-C₆-Alkoxy, C₁-C₆-Alkylthio und NR^{A}R^{B};
R^{A}, R^{B} Wasserstoff und C₁-C₆-Alkyl;
- R²: C₁-C₁₂-Alkoxy-C₁-C₁₂-alkyl, Phenoxy-C₁-C₁₂-alkyl, C₁-C₁₂-Alkylthio-C₁-C₁₂-alkyl und Phenylthio-C₁-C₁₂-alkyl, in welchen Gruppen die Kohlenstoffketten durch eine bis drei Gruppen R^{a} substituiert und die Phenylringe durch einen bis fünf Substituenten aus C₁-C₆-Alkyl oder einer Gruppe R^{a} substituiert sein können;
- R³: Wasserstoff und C₁-C₆-Alkyl;
- A: N und C-R^{A}.

Außerdem betrifft die Erfindung Verfahren zur Herstellung dieser Verbindungen, sie enthaltende Mittel sowie ihre Verwendung zur Bekämpfung von pflanzenpathogenen Schadpilzen.

In GB 1 148 629 werden 5,6-Dialkyl-7-amino-triazolopyrimidine allgemein vorgeschlagen. Aus EP-A 141 317 sind einzelne fungizid wirksame 5,6-Dialkyl-7-amino-azolopyrimidine bekannt. Ihre Wirkung ist jedoch in vielen Fällen nicht zufriedenstellend. Davon ausgehend, liegt der vorliegenden Erfindung die Aufgabe zugrunde, Verbindungen mit verbesserter Wirkung und/oder verbreitertem Wirkungsspektrum bereitzustellen.

Demgemäss wurden die eingangs definierten Verbindungen gefunden. Des weiteren wurden Verfahren und Zwischenprodukte zu ihrer Herstellung, sie enthaltende Mittel sowie Verfahren zur Bekämpfung von Schadpilzen unter Verwendung der Verbindungen I gefunden.

Die Verbindungen der Formel I unterscheiden sich von den aus den oben genannten Schriften durch die spezielle Ausgestaltung des Substituenten in der 5-Position des Triazolopyrimidin-Gerüstes.

Die Verbindungen der Formel I weisen eine gegenüber den bekannten Verbindungen erhöhte Wirksamkeit gegen Schadpilze auf.

Die erfindungsgemäßen Verbindungen können auf verschiedenen Wegen erhalten werden. Vorteilhaft werden die erfindungsgemäßen Verbindungen erhalten, indem man substituierte β-Ketoestern der Formel II mit 3-Amino-1,2,4-triazol oder-pyrazol der Formel III zu 7-Hydroxyazolopyrimidinen der Formel IV umsetzt. Die Gruppen R¹ und R² in Formeln II und IV haben die Bedeutungen wie für Formel I und die Gruppe R in Formel II bedeutet C₁-C₄-Alkyl, aus praktischen Gründen ist Methyl, Ethyl oder Propyl darin bevorzugt. 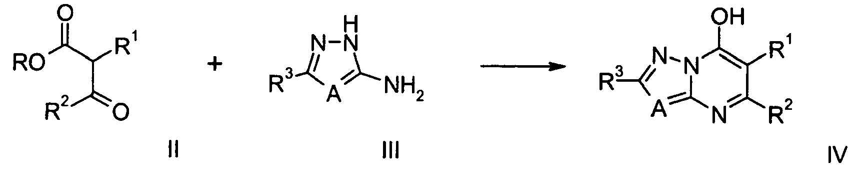 Die Umsetzung der substituierten β-Ketoester der Formel II mit den Aminoazolen der Formel III kann in Gegenwart oder Abwesenheit von Lösungsmitteln durchgeführt werden. Vorteilhaft ist es, solche Lösungsmittel zu verwenden, gegenüber denen die Einsatzstoffe weitgehend inert sind und in denen sie ganz oder teilweise löslich sind. Als Lösungsmittel kommen insbesondere Alkohole wie Ethanol, Propanole, Butanole, Glykole oder Glykolmonoether, Diethylenglykole oder deren Monoether, aromatische Kohlenwasserstoffe, wie Toluol, Benzol oder Mesitylen, Amide wie Dimethylformamid, Diethylformamid, Dibutylformamid, N,N-Dimethylacetamid, niedere Alkansäuren wie Ameisensäure, Essigsäure, Propionsäure oder Basen, wie Alkalimetall- und Erdalkalimetallhydroxide, Alkalimetall- und Erdalkalimetalloxide, Alkalimetall- und Erdalkalimetallhydride, Alkalimetallamide, Alkalimetall- und Erdalkalimetallcarbonate sowie Alkalimetallhydrogencarbonate, metallorganische Verbindungen, insbesondere Alkalimetallalkyle, Alkylmagnesiumhalogenide sowie Alkalimetall- und Erdalkalimetallalkoholate und Dimethoxymagnesium, außerdem organische Basen, z.B. tertiäre Amine wie Trimethylamin, Triethylamin, Tri-isopropylethylamin, Tributylamin und N-Methylpiperidin, N-Methylmorpholin, Pyridin, substituierte Pyridine wie Collidin, Lutidin und 4-Dimethylaminopyridin sowie bicyclische Amine und Mischungen dieser Lösungsmittel mit Wasser in Frage. Als Katalysatoren kommen Basen, wie voranstehend genannt, oder Säuren, wie Sulfonsäuren oder Mineralsäuren in Frage. Besonders bevorzugt wird die Umsetzung ohne Lösungsmittel oder in Chlorbenzol, Xylol, Dimethylsulfoxid, N-Methylpyrrolidon durchgeführt. Besonders bevorzugte Basen sind tertiäre Amine wie Tri-isopropylamin, Tributylamin, N-Methylmorpholin oder N-Methylpiperidin. Die Temperaturen liegen zwischen 50 und 300°C, vorzugsweise bei 50 bis 180°C, wenn in Lösung gearbeitet wird [vgl. EP-A 770 615; Adv. Het. Chem. Bd. 57, S. 81ff. (1993)].

Die Basen werden im allgemeinen in katalytischen Mengen eingesetzt, sie können aber auch äquimolar, im Überschuss oder gegebenenfalls als Lösungsmittel verwendet werden. 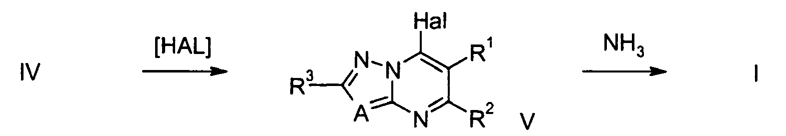 Die so erhaltenen Kondensationsprodukte der Formel IV fallen aus den Reaktionslösungen meist in reiner Form aus und werden nach dem Waschen mit dem gleichen Lösungsmittel oder mit Wasser und anschließendem Trocknen mit Halogenierungsmitteln, insbesondere Chlorierungs- oder Bromierungsmittel zu den Verbindungen der Formel V, in der Hal für Chlor oder Brom, insbesondere für Chlor steht, umgesetzt. Bevorzugt erfolgt die Umsetzung mit Chlorierungsmitteln, wie Phosphoroxychlorid, Thionylchlorid oder Sulfurylchlorid bei 50°C bis 150°C vorzugsweise in überschüssigem Phosphoroxitrichlorid bei Rückflusstemperatur. Nach dem Verdampfen des überschüssigen Phosphoroxitrichlorids wird der Rückstand mit Eiswasser gegebenenfalls unter Zusatz eines mit Wasser nicht mischbaren Lösungsmittels behandelt. Das aus der getrockneten organischen Phase gegebenenfalls nach Verdampfung des inerten Lösungsmittels isolierte Chlorierungsprodukt ist meist sehr rein und wird anschließend mit Ammoniak in inerten Lösungsmitteln bei 100°C bis 200°C zu den 7-Amino-azolo[1,5-a]-pyrimidinen umgesetzt. Die Reaktion wird vorzugsweise mit 1- bis 10-molarem Überschuss an Ammoniak unter Druck von 1 bis 100 bar durchgeführt.

Die neuen 7-Amino-azolo[1,5-a]-pyrimidine werden gegebenenfalls nach Verdampfen des Lösungsmittels durch Digerieren in Wasser als kristalline Verbindungen isoliert.

Die β-Ketoester der Formel II können hergestellt werden wie in Organic Synthesis Coll. Vol. 1, S. 248 beschrieben, bzw. sind kommerziell erhältlich.

Alternativ können die neuen Verbindungen der Formel 1 erhalten werden, indem man substituierte Acylcyanide der Formel VI, in der R¹ und R² die oben angegebenen Bedeutungen haben, mit 3-Amino-1,2,4-triazol der Formel III umsetzt.

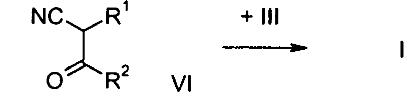

Die Umsetzung kann in Gegenwart oder Abwesenheit von Lösungsmitteln durchgeführt werden. Vorteilhaft ist es, solche Lösungsmittel zu verwenden, gegenüber denen die Einsatzstoffe weitgehend inert sind und in denen sie ganz oder teilweise löslich sind. Als Lösungsmittel kommen insbesondere Alkohole wie Ethanol, Propanole, Butanole, Glykole oder Glykolmonoether, Diethylenglykole oder deren Monoether, aromatische Kohlenwasserstoffe, wie Toluol, Benzol oder Mesitylen, Amide wie Dimethylformamid, Diethylformamid, Dibutylformamid, N,N-Dimethylacetamid, niedere Alkansäuren wie Ameisensäure, Essigsäure, Propionsäure oder Basen, wie voranstehend genannt, und Mischungen dieser Lösungsmittel mit Wasser in Frage. Die Umsetzungstemperaturen liegen zwischen 50 und 300°C, vorzugsweise bei 50 bis 150°C, wenn in Lösung gearbeitet wird.

Die neuen 7-Amino-triazolo[1,5-a]-pyrimidine werden gegebenenfalls nach Verdampfen des Lösungsmittels oder Verdünnen mit Wasser als kristalline Verbindungen isoliert.

Die für die Herstellung der 7-Amino-azolo[1,5-a]-pyrimidine benötigten substituierten Alkylcyanide der Formel VI sind teilweise bekannt oder können nach bekannten Methoden aus Alkylcyaniden und Carbonsäureestern mit starken Basen, z.B. Alkalihydriden, Alkalimetallalkoholaten, Alkaliamiden oder Metallalkylen, hergestellt werden [vgl.: J. Amer. Chem. Soc. Bd. 73, (1951) S. 3766].

Sofern einzelne Verbindungen 1 nicht auf den voranstehend beschriebenen Wegen zugänglich sind, können sie durch Derivatisierung anderer Verbindungen I hergestellt werden.

Sofern bei der Synthese Isomerengemische anfallen, ist im allgemeinen jedoch eine Trennung nicht unbedingt erforderlich, da sich die einzelnen Isomere teilweise während der Aufbereitung für die Anwendung oder bei der Anwendung (z.B. unter Licht-, Säure- oder Baseneinwirkung) ineinander umwandeln können. Entsprechende Umwandlungen können auch nach der Anwendung, beispielsweise bei der Behandlung von Pflanzen in der behandelten Pflanze oder im zu bekämpfenden Schadpilz erfolgen.

Bei den in den vorstehenden Formeln angegebenen Definitionen der Symbole wurden Sammelbegriffe verwendet, die allgemein repräsentativ für die folgenden Substituenten stehen:
Halogen: Fluor, Chlor, Brom und Jod;
Alkyl: gesättigte, geradkettige oder ein- oder zweifach verzweigte Kohlenwasserstoffreste mit 1 bis 4, oder 5 bis 12 Kohlenstoffatomen, z.B. C₁-C₆-Alkyl wie Methyl, Ethyl, Propyl, 1-Methylethyl, Butyl, 1-Methyl-propyl, 2-Methylpropyl, 1,1-Dimethylethyl, n-Pentyl, 1-Methylbutyl, 2-Methylbutyl, 3-Methylbutyl, 2,2-Di-methylpropyl, 1-Ethylpropyl, Hexyl, 1,1-Dimethylpropyl, 1,2-Dimethylpropyl, 1-Methylpentyl, 2-Methylpentyl, 3-Methylpentyl, 4-Methylpentyl, 1,1-Dimethylbutyl, 1,2-Dimethylbutyl, 1,3-Dimethylbutyl, 2,2-Dimethylbutyl, 2,3-Dimethylbutyl, 3,3-Dimethylbutyl, 1-Ethylbutyl, 2-Ethylbutyl, 1,1,2-Trimethylpropyl, 1,2,2-Trimethylpropyl, 1-Ethyl-1-methylpropyl und 1-Ethyl-2-methylpropyl;
Halogenmethyl: Methylgruppe, in der teilweise oder vollständig die Wasserstoffatome durch Halogenatome wie vorstehend genannt ersetzt sein können: insbesondere Chlormethyl, Brommethyl, Dichlormethyl, Trichlormethyl, Fluormethyl, Difluormethyl, Trifluormethyl, Chlorfluormethyl, Dichlorfluormethyl, Chlordifluormethyl;
Cycloalkyl: mono- oder bicyclische, gesättigte Kohlenwasserstoffgruppen mit 3 bis 6 Kohlenstoffringgliedern, wie Cyclopropyl, Cyclobutyl, Cyclopentyl und Cyclohexyl;
Alkoxyalkyl: gesättigte, geradkettige oder ein-, zwei- oder dreifach verzweigte Kohlenwasserstoffkette, die durch ein Sauerstoffatom unterbrochen ist, z. B. C₅-C₁₂-Alkoxyalkyl: Kohlenwasserstoffkette wie voranstehend beschreiben mit 5 bis 12 Kohlenstoffatomen, die durch ein Sauerstoffatom an beliebiger Stelle unterbrochen sein kann, wie Propoxy-ethyl, Butoxy-ethyl, Pentoxy-ethyl, Hexyloxy-ethyl, Heptyloxy-ethyl, Octyloxyethyl, Nonyloxy-ethyl, 3-(3-Ethyl-hexyloxy)-ethyl, 3-(2,4,4-Trimethyl-pentyloxy)-ethyl, 3-(1-Ethyl-3-methyl-butoxy)-ethyl, Ethoxy-propyl, Propoxy-propyl, Butoxy-propyl, Pentoxy-propyl, Hexyloxy-propyl, Heptyloxy-propyl, Octyloxy-propyl, Nonyloxy-propyl, 3-(3-Ethyl-hexyloxy)-propyl, 3-(2,4,4-Trimethyl-pentyloxy)-propyl, 3-(1-Ethyl-3-methylbutoxy)-propyl, Ethoxy-butyl, Propoxy-butyl, Butoxy-butyl, Pentoxy-butyl, Hexyloxybutyl, Heptyloxy-butyl, Octyloxy-butyl, Nonyloxy-butyl, 3-(3-Ethyl-hexyloxy)-butyl, 3-(2,4,4-Trimethyl-pentyloxy)-butyl, 3-(1-Ethyl-3-methyl-butoxy)-butyl, Methoxy-pentyl, Ethoxy-pentyl, Propoxy-pentyl, Butoxy-pentyl, Pentoxy-pentyl, Hexyloxy-pentyl, Heptyloxy-pentyl, 3-(3-Methyl-hexyloxy)-pentyl, 3-(2,4-Dimethyl-pentyloxy)-pentyl, 3-(1-Ethyl-3-methyl-butoxy)-pentyl;

In dem Umfang der vorliegenden Erfindung sind die (R)- und (S)-Isomere und die Razemate von Verbindungen der Formel I eingeschlossen, die chirale Zentren aufweisen.

Im Hinblick auf ihre bestimmungsgemäße Verwendung der Azolopyrimidine der Formel I sind die folgenden Bedeutungen der Substituenten, und zwar jeweils für sich allein oder in Kombination, besonders bevorzugt:

Verbindungen I werden bevorzugt, in denen die Gruppe R¹ maximal 12 Kohlenstoffatome aufweist.

Die Alkylgruppen in R¹ in Formel I stellen bevorzugt unverzweigte oder ein-, zwei-, drei- oder mehrfach verzweigte, insbesondere eine unverzweigte C₁-C₁₂-Alkylgruppe dar.

Daneben werden Verbindungen der Formel I bevorzugt, die in R¹ am α-Kohlenstoffatom eine Verzweigung aufweisen. Sie werden durch Formel la beschrieben: 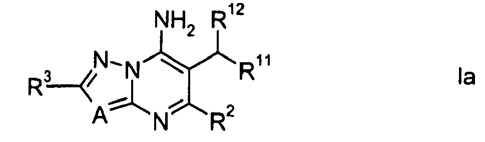 in der R¹¹ C₃-C₁₀-Alkyl oder C₅-C₁₀-Alkoxyalkyl und R¹² C₁-C₄-Alkyl, insbesondere Methyl, bedeuten, wobei R¹¹ und R¹² gemeinsam nicht mehr als 12 Kohlenstoffatome aufweisen und unsubstituiert sind oder wie R¹ in Formel I substituiert sein können und die anderen Variablen wie für Formel I definiert sind.

Sofern R¹ eine durch Cyano substituierte Alkylgruppe darstellt, steht die Cyanogruppe bevorzugt am endständigen Kohlenstoffatom.

Sofern R¹ eine durch Halogen substituierte Alkylgruppe darstellt, liegt die Halogenierung bevorzugt am α- oder am Ω-Kohlenstoffatom vor.

In einer weiteren bevorzugten Ausgestaltung steht R¹ für eine durch Hydroxy substituierte Alkylgruppe.

Bevorzugt sind Verbindungen I, in denen R¹ für eine unverzweigte oder ein-, zwei-, drei- oder mehrfach verzweigte C₅-C₁₂-Alkylgruppe oder C₅-C₁₀-Alkoxypropylgruppe steht, die keine weiteren Substituenten trägt.

Besonders bevorzugt sind Verbindungen I, in denen R¹ für n-Pentyl, 1-Methylbutyl, 2-Methylbutyl, 3-Methylbutyl, 2,2-Di-methylpropyl, 1-Ethylpropyl, n-Hexyl, 1,1-Dimethylpropyl, 1,2-Dimethylpropyl, 1-Methylpentyl, 2-Methylpentyl, 3-Methylpentyl, 4-Methylpentyl, 1,1-Dimethylbutyl, 1,2-Dimethylbutyl, 1,3-Dimethylbutyl, 2,2-Dimethylbutyl, 2,3-Dimethylbutyl, 3,3-Dimethylbutyl, 1-Ethylbutyl, 2-Ethylbutyl, 1,1,2-Trimethylpropyl, 1,2,2-Trimethylpropyl, 1-Ethyl-1-methylpropyl oder 1-Ethyl-2-methylpropyl steht.

Daneben sind Verbindungen der Formel 1 bevorzugt, in denen R¹ für n-Heptyl, 1-Methylhexyl, n-Octyl, 1-Methylheptyl, n-Nonyl, 1-Methyloctyl, 3,5,5-Trimethylhexyl, n-Decyl, 1-Methylnonyl, n-Undecyl, 1-Methyldecyl, n-Dodecyl und 1-Methylundecyl steht.

In einer weiteren bevorzugten Ausgestaltung der erfindungsgemäßen Verbindungen steht R¹ für Methoxy-n-propyl, Ethoxy-n-propyl, n-Propoxy-n-propyl, n-Butoxy-n-propyl, n-Pentyloxy-n-propyl, n-Hexyloxypropyl, n-Heptyloxy-n-propyl, n-Octyloxy-n-propyl, n-Nonyloxy-n-propyl oder n-Decyloxy-n-propyl.

In einer bevorzugten Ausgestaltung der erfindungsgemäßen Verbindungen I steht R² für C₁-C₁₂-Alkoxy-C₁-C₁₂-alkyl, insbesondere für C₁-C₁₂-Alkoxymethyl.

In einer weiteren bevorzugten Ausgestaltung steht R² für Methoxy-C₁-C₁₂-alkyl, insbesondere für Methoxymethyl.

In einer bevorzugten Ausführungsform der erfindungsgemäßen Verbindungen I steht A für ein Stickstoffatom.

In einer anderen Ausgestaltung der Verbindungen I steht A für CR⁴, insbesondere für CH.

Daneben sind auch Verbindungen I bevorzugt, in denen R³ Wasserstoff bedeutet.

Eine besonders bevorzugte Ausgestaltung der erfindungsgemäßen Verbindungen der Formel I sind solche der Formel I.A: 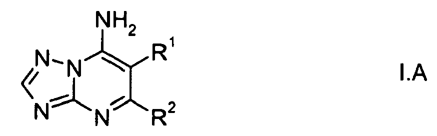 in der R¹ und R² wie für Formel I definiert sind, wobei R¹ insbesondere für C₁-C₁₂-Alkyl und R² insbesondere für C₁-C₁₂-Alkoxymethyl, bevorzugt Methoxymethyl steht.

Insbesondere sind im Hinblick auf ihre Verwendung die in den folgenden Tabellen zusammengestellten Verbindungen I bevorzugt. Die in den Tabellen für einen Substituenten genannten Gruppen stellen außerdem für sich betrachtet, unabhängig von der Kombination, in der sie genannt sind, eine besonders bevorzugte Ausgestaltung des betreffenden Substituenten dar.

### Tabelle 1

Verbindungen der Formel I.A, in denen R¹ für eine Verbindung jeweils einer Zeile der Tabelle A entspricht und R² Methoxymethyl bedeutet

### Tabelle 2

Verbindungen der Formel I.A, in denen R¹ für eine Verbindung jeweils einer Zeile der Tabelle A entspricht und R² Ethoxymethyl bedeutet

### Tabelle 3

Verbindungen der Formel I.A, in denen R¹ für eine Verbindung jeweils einer Zeile der Tabelle A entspricht und R² n-Propoxymethyl bedeutet

### Tabelle 4

Verbindungen der Formel I.A, in denen R¹ für eine Verbindung jeweils einer Zeile der Tabelle A entspricht und R² Methoxyethyl bedeutet

### Tabelle 5

Verbindungen der Formel I.A, in denen R¹ für eine Verbindung jeweils einer Zeile der Tabelle A entspricht und R² Ethoxyethyl bedeutet

### Tabelle 6

Verbindungen der Formel I.A, in denen R¹ für eine Verbindung jeweils einer Zeile der Tabelle A entspricht und R² n-Propoxyethyl bedeutet.

### Tabelle 7

Verbindungen der Formel I.A, in denen R¹ für eine Verbindung jeweils einer Zeile der Tabelle A entspricht und R² 3-Methoxy-n-propyl bedeutet

### Tabelle 8

Verbindungen der Formel I.A, in denen R¹ für eine Verbindung jeweils einer Zeile der Tabelle A entspricht und R² 3-Ethoxy-n-propyl bedeutet

### Tabelle 9

Verbindungen der Formel I.A, in denen R¹ für eine Verbindung jeweils einer Zeile der Tabelle A entspricht und R² 3-n-Propoxy-n-propyl bedeutet

### Tabelle 10

Verbindungen der Formel I, in denen R¹ für eine Verbindung jeweils einer Zeile der Tabelle A entspricht, R² Methoxymethyl, R³ Wasserstoff und A CH bedeutet

### Tabelle 11

Verbindungen der Formel I, in denen R¹ für eine Verbindung jeweils einer Zeile der Tabelle A entspricht, R² Ethoxymethyl, R³ Wasserstoff und A CH bedeutet

### Tabelle 12

Verbindungen der Formel I, in denen R¹ für eine Verbindung jeweils einer Zeile der Tabelle A entspricht, R² n-Propoxymethyl, R³ Wasserstoff und A CH bedeutet

### Tabelle 13

Verbindungen der Formel I, in denen R¹ für eine Verbindung jeweils einer Zeile der Tabelle A entspricht, R² Methoxyethyl, R³ Wasserstoff und A CH bedeutet

### Tabelle 14

Verbindungen der Formel I, in denen R¹ für eine Verbindung jeweils einer Zeile der Tabelle A entspricht, R² Ethoxyethyl, R³ Wasserstoff und A CH bedeutet

### Tabelle 15

Verbindungen der Formel I, in denen R¹ für eine Verbindung jeweils einer Zeile der Tabelle A entspricht, R² n-Propoxyethyl, R³ Wasserstoff und A CH bedeutet.

### Tabelle 16

Verbindungen der Formel I, in denen R¹ für eine Verbindung jeweils einer Zeile der Tabelle A entspricht, R² 3-Methoxy-n-propyl, R³ Wasserstoff und A CH bedeutet

### Tabelle 17

Verbindungen der Formel I, in denen R¹ für eine Verbindung jeweils einer Zeile der Tabelle A entspricht, R² 3-Ethoxy-n-propyl, R³ Wasserstoff und A CH bedeutet

### Tabelle 18

Verbindungen der Formel I, in denen R¹ für eine Verbindung jeweils einer Zeile der Tabelle A entspricht, R² 3-n-Propoxy-n-propyl, R³ Wasserstoff und A CH bedeutet

### Tabelle 19

Verbindungen der Formel I, in denen R¹ für eine Verbindung jeweils einer Zeile der Tabelle A entspricht, R² Methoxymethyl, R³ CH₃ und A CH bedeutet

### Tabelle 20

Verbindungen der Formel I.A, in denen R¹ für eine Verbindung jeweils einer Zeile der Tabelle A entspricht, R² Ethoxymethyl, R³ CH₃ und A CH bedeutet

### Tabelle 21

Verbindungen der Formel I.A, in denen R¹ für eine Verbindung jeweils einer Zeile der Tabelle A entspricht, R² n-Propoxymethyl, R³ CH₃ und A CH bedeutet

### Tabelle 22

Verbindungen der Formel I.A, in denen R¹ für eine Verbindung jeweils einer Zeile der Tabelle A entspricht, R² Methoxyethyl, R³ CH₃ und A CH bedeutet

### Tabelle 23

Verbindungen der Formel LA, in denen R¹ für eine Verbindung jeweils einer Zeile der Tabelle A entspricht, R² Ethoxyethyl, R³ CH₃ und A CH bedeutet

### Tabelle 24

Verbindungen der Formel I.A, in denen R¹ für eine Verbindung jeweils einer Zeile der Tabelle A entspricht, R² n-Propoxyethyl, R³ CH₃ und A CH bedeutet.

### Tabelle 25

Verbindungen der Formel I.A, in denen R¹ für eine Verbindung jeweils einer Zeile der Tabelle A entspricht, R² 3-Methoxy-n-propyl, R³ CH₃ und A CH bedeutet

### Tabelle 26

Verbindungen der Formel I.A, in denen R¹ für eine Verbindung jeweils einer Zeile der Tabelle A entspricht, R² 3-Ethoxy-n-propyl, R³ CH₃ und A CH bedeutet

### Tabelle 27

Verbindungen der Formel I.A, in denen R¹ für eine Verbindung jeweils einer Zeile der Tabelle A entspricht, R² 3-n-Propoxy-n-propyl, R³ CH₃ und A CH bedeutet

**Tabelle A**

| **Nr.** | **R¹** |
|---|---|
| A-1 | CH₂CH₂CH₂CH₂CH₃ |
| A-2 | CH(CH₃)CH₂CH₂CH₃ |
| A-3 | CH₂CH(CH₃)CH₂CH₃ |
| A-4 | CH₂CH₂CH(CH₃)CH₃ |
| A-5 | CH₂CH₂CH(CH₃)₂ |
| A-6 | CH(CH₃)CH(CH₃)CH₃ |
| A-7 | CH(CH₃)CH(CH₃)₂ |
| A-8 | CH₂C(CH₃)₃ |
| A-9 | CH₂CH₂CH₂CH₂CH₂CH₃ |
| A-10 | CH(CH₃)CH₂CH₂CH₂CH₃ |
| A-11 | CH₂CH(CH₃)CH₂CH₂CH₃ |
| A-12 | CH₂CH₂CH(CH₃)CH₂CH₃ |
| A-13 | CH₂CH₂CH(CH₃)₂CH₂ |
| A-14 | CH₂CH₂CH₂CH(CH₃)₂ |
| A-15 | CH(CH₃)CH(CH₃)CH₂CH₃ |
| A-16 | CH(CH₃)CH₂CH(CH₃)₂ |
| A-17 | CH₂CH₂C(CH₃)₃ |
| A-18 | CH(CH₃)CH₂CH(CH₃)CH₃ |
| A-19 | CH₂CH₂CH₂CH₂CH₂CH₂CH₃ |
| A-20 | CH(CH₃)CH₂CH₂CH₂CH₂CH₃ |
| A-21 | CH₂CH(CH₃)CH₂CH₂CH₂CH₃ |
| A-22 | CH₂CH₂CH(CH₃)CH₂CH₂CH₃ |
| A-23 | CH₂CH₂CH₂CH(CH₃)CH₂CH₃ |
| A-24 | CH₂CH₂CH₂CH₂CH(CH₃)CH₃ |
| A-25 | CH₂CH₂CH₂CH₂CH(CH₃)₂ |
| A-26 | CH(CH₃)CH(CH₃)CH₂CH₂CH₃ |
| A-27 | CH₂CH(CH₃)CH(CH₃)CH₂CH₃ |
| A-28 | CH₂CH₂CH₂C(CH₃)₃ |
| A-29 | CH(CH₃)CH₂CH(CH₃)CH₂CH₃ |
| A-30 | CH₂CH(CH₃)CH(CH₃)CH₂CH₃ |
| A-31 | CH(CH₃)CH₂CH₂CH(CH₃)CH₃ |
| A-32 | CH₂CH₂CH₂CH₂CH₂CH₂CH₂CH₃ |
| A-33 | CH(CH₃)CH₂CH₂CH₂CH₂CH₂CH₃ |
| A-34 | CH₂CH(CH₃)CH₂CH₂CH₂CH₂CH₃ |
| A-35 | CH₂CH₂CH(CH₃)CH₂CH₂CH₂CH₃ |
| A-36 | CH₂CH₂CH₂CH(CH₃)CH₂CH₂CH₃ |
| A-37 | CH₂CH₂CH₂CH₂CH(CH₃)CH₂CH₃ |
| A-38 | CH₂CH₂CH₂CH₂CH₂CH(CH₃)₂ |
| A-39 | CH₂CH₂CH₂CH₂C(CH₃)₃ |
| A-40 | CH(CH₃)CH(CH₃)CH₂CH₂CH₂CH₃ |
| A-41 | CH₂CH(CH₃)CH(CH₃)CH₂CH₂CH₃ |
| A-42 | CH₂CH₂CH₂C(CH₃)₂CH₂CH₃ |
| A-43 | CH(CH₃)CH₂CH(CH₃)CH₂CH₂CH₃ |
| A-44 | CH₂CH(CH₃)CH(CH₃)CH₂CH₂CH₃ |
| A-45 | CH(CH₃)CH₂CH₂CH(CH₃)CH₂CH₃ |
| A-46 | CH(CH₃)CH₂CH₂CH₂CH(CH₃)₂ |
| A-47 | CH₂CH₂CH(CH₃)CH₂C(CH₃)₃ |
| A-48 | CH₂CH₂CH₂CH₂CH₂CH₂CH₂CH₂CH₃ |
| A-49 | CH(CH₃)CH₂CH₂CH₂CH₂CH₂CH₂CH₃ |
| A-50 | CH₂CH(CH₃)CH₂CH₂CH₂CH₂CH₂CH₃ |
| A-51 | CH₂CH₂CH(CH₃)CH₂CH₂CH₂CH₂CH₃ |
| A-52 | CH₂CH₂CH₂CH(CH₃)CH₂CH₂CH₂CH₃ |
| A-53 | CH₂CH₂CH₂CH₂CH(CH₃)CH₂CH₂CH₃ |
| A-54 | CH₂CH₂CH₂CH₂CH₂CH₂C(CH₃)₃ |
| A-55 | CH(CH₃)CH(CH₃)CH₂CH₂CH₂CH₂CH₃ |
| A-56 | CH₂CH(CH₃)CH(CH₃)CH₂CH₂CH₂CH₃ |
| A-57 | CH₂CH₂CH₂C(CH₃)₂CH₂CH₂CH₃ |
| A-58 | CH(CH₃)CH₂CH(CH₃)CH₂CH₂CH₂CH₃ |
| A-59 | CH₂CH(CH₃)CH(CH₃)CH₂CH₂CH₂CH₃ |
| A-60 | CH(CH₃)CH₂CH₂CH(CH₃)CH₂CH₂CH₃ |
| A-61 | CH(CH₃)CH₂CH₂CH₂C(CH₃)₃ |
| A-62 | CH₂CH(CH₃)CH₂CH₂CH(CH₃)₃ |
| A-63 | CH(CH₃)CH₂CH₂CH₂CH₂CH(CH₃)₂ |
| A-64 | CH₂CH(CH₃)CH₂CH₂CH₂CH(CH₃)₂ |
| A-65 | CH₂CH₂CH₂CH₂CH₂CH₂CH₂CH₂CH₂CH₃ |
| A-66 | CH(CH₃)CH₂CH₂CH₂CH₂CH₂CH₂CH₂CH₃ |
| A-67 | CH₂CH(CH₃)CH₂CH₂CH₂CH₂CH₂CH₂CH₃ |
| A-68 | CH₂CH₂CH(CH₃)CH₂CH₂CH₂CH₂CH₂CH₃ |
| A-69 | CH₂CH₂CH(CH₃)CH₂CH₂CH₂CH₂CH₂ |
| A-70 | CH₂CH₂CH₂CH(CH₃)CH₂CH₂CH₂CH₃ |
| A-71 | CH₂CH₂CH₂CH₂CH₂CH₂C(CH₃)₃ |
| A-72 | CH(CH₃)CH(CH₃)CH₂CH₂CH₂CH₂CH₂CH₃ |
| A-73 | CH₂CH(CH₃)CH(CH₃)CH₂CH₂CH₂CH₂CH₃ |
| A-74 | CH₂CH₂CH₂C(CH₃)₂CH₂CH₂CH₂CH₃ |
| A-75 | CH(CH₃)CH₂CH(CH₃)CH₂CH₂CH₂CH₂CH₃ |
| A-76 | CH₂CH(CH₃)CH(CH₃)CH₂CH₂CH₂CH₂CH₃ |
| A-77 | CH(CH₃)CH₂CH₂CH(CH₃)CH₂CH₂CH₂CH₃ |
| A-78 | CH(CH₃)CH₂CH₂CH₂CH(CH₃)CH₂CH₂CH₃ |
| A-79 | CH(CH₃)CH₂CH₂CH₂CH₂CH(CH₃)CH₂CH₃ |
| A-80 | CH(CH₃)CH₂CH₂CH₂CH₂CH₂CH(CH₃)₂ |
| A-81 | CH(CH₃)CH₂CH₂CH₂CH₂CH₂C(CH₃)CH₃ |
| A-82 | CH₂CH(CH₃)CH₂CH₂CH₂CH₂CH(CH₃)CH₃ |
| A-83 | CH(CH₃)CH₂CH₂CH₂CH₂C(CH₃)₃ |
| A-84 | CH₂CH(CH₃)CH₂CH₂CH₂C(CH₃)₃ |
| A-85 | CH₂CH₂CH₂CH₂CH₂CH₂CH₂CH₂CH₂CH₂CH₃ |
| A-86 | CH(CH₃)CH₂CH₂CH₂CH₂CH₂CH₂CH₂CH₂CH₃ |
| A-87 | CH₂CH(CH₃)CH₂CH₂CH₂CH₂CH₂CH₂CH₂CH₃ |
| A-88 | CH₂CH₂CH(CH₃)CH₂CH₂CH₂CH₂CH₂CH₂CH₃ |
| A-89 | CH₂CH₂CH₂CH(CH₃)CH₂CH₂CH₂CH₂CH₂CH₃ |
| A-90 | CH₂CH₂CH₂CH₂CH(CH₃)CH₂CH₂CH₂CH₂CH₃ |
| A-91 | CH₂CH₂CH₂CH₂CH₂CH₂CH₂C(CH₃)₃ |
| A-92 | CH(CH₃)CH(CH₃)CH₂CH₂CH₂CH₂CH₂CH₂CH₃ |
| A-93 | CH₂CH(CH₃)CH(CH₃)CH₂CH₂CH₂CH₂CH₂CH₃ |
| A-94 | CH₂CH₂CH₂C(CH₃)₂CH₂CH₂CH₂CH₂CH₃ |
| A-95 | CH(CH₃)CH₂CH(CH₃)CH₂CH₂CH₂CH₂CH₂CH₃ |
| A-96 | CH₂CH(CH₃)CH(CH₃)CH₂CH₂CH₂CH₂CH₂CH₃ |
| A-97 | CH(CH₃)CH₂CH₂CH(CH₃)CH₂CH₂CH₂CH₂CH₃ |
| A-98 | CH(CH₃)CH₂CH₂CH₂CH(CH₃)CH₂CH₂CH₂CH₃ |
| A-99 | CH(CH₃)CH₂CH₂CH₂CH₂CH(CH₃)CH₂CH₂CH₃ |
| A-100 | CH(CH₃)CH₂CH₂CH₂CH₂CH₂CH(CH₃)CH₂CH₃ |
| A-101 | CH(CH₃)CH₂CH₂CH_{z}CH_{Z}CH_{z}CH₂CH(CH₃)_{z} |
| A-102 | CH₂CH(CH₃)CH₂CH₂CH₂CH₂CH(CH₃)CH₂CH₃ |
| A-103 | CH₂CH(CH₃)CH₂CH₂CH₂CH₂CH(CH₃)CH₂CH₃ |
| A-104 | CH₂CH₂CH(CH₃)CH₂CH₂CH₂CH₂CH(CH₃)₂ |
| A-105 | CH₂CH(CH₃)CH₂CH₂CH₂CH₂C(CH₃)₃ |
| A-106 | CH₂CH₂CH₂CH₂CH₂CH₂CH₂CH₂CH₂CH₂CH₂CH₃ |
| A-107 | CH(CH₃)CH₂CH₂ CH₂CH₂CH₂CH₂CH₂CH₂CH₂CH₃ |
| A-108 | CH₂CH(CH₃)CH₂CH₂CH₂CH₂CH₂CH₂CH₂CH₂CH₃ |
| A-109 | CH₂CH₂CH(CH₃)CH₂CH₂CH₂CH₂CH₂CH₂CH₂CH₃ |
| A-110 | CH₂ CH₂CH₂CH(CH₃)CH₂CH₂CH₂CH₂CH₂CH₂CH₂ |
| A-111 | CH₂CH₂CH₂CH₂CH(CH₃)CH₂CH₂CH₂CH₂CH₂CH₃ |
| A-112 | CH₂CH₂CH₂CH₂CH₂CH(CH₃)CH₂CH₂CH₂CH₂CH₃ |
| A-113 | CH₂CH₂CH₂CH₂CH₂CH₂CH(CH₃)CH₂CH₂CH₂CH₃ |
| A-114 | CH₂CH₂CH₂CH₂CH₂CH₂CH₂CH(CH₃)CH₂CH₂CH₃ |
| A-115 | CH₂CH₂CH₂CH₂CH₂CH₂CH₂CH₂CH(CH₃)CH₂CH₃ |
| A-116 | CH₂CH₂CH₂CH₂CH₂CH₂CH₂CH₂CH₂CH(CH₃)₂ |
| A-117 | CH(CH₃)CH(CH₃)CH₂CH₂CH₂CH₂CH₂CH₂CH₂CH₃ |
| A-118 | CH₂CH(CH₃)CH(CH₃)CH₂CH₂CH₂CH₂CH₂CH₂CH₃ |
| A-119 | CH₂CH₂CH₂C(CH₃)₂CH₂CH₂CH₂CH₂CH₂CH₃ |
| A-120 | CH₂CH₂CH₂CH₂CH(CH₃)CH₂CH₂CH₂CH₂CH₂CH₃ |
| A-121 | CH(CH₃)CH₂CH(CH₃)CH₂CH₂CH₂CH₂ CH₂CH₂CH₃ |
| A-122 | CH(CH₃)CH₂CH₂CH(CH₃)CH₂CH₂CH₂ CH₂CH₂CH₃ |
| A-123 | CH(CH₃)CH₂CH₂CH₂CH(CH₃)CH₂CH₂CH₂CH₂CH₃ |
| A-124 | CH(CH₃)CH₂CH₂CH₂CH₂CH(CH₃)CH₂CH₂CH₂CH₃ |
| A-125 | CH(CH₃)CH₂CH₂CH₂CH₂CH₂CH(CH₃)CH₂CH₂CH₃ |
| A-126 | CH(CH₃)CH₂CH₂CH₂CH₂CH₂CH₂CH(CH₃)CH₂CH₃ |
| A-127 | CH₂CH(CH₃)CH₂CH₂CH₂CH₂CH(CH₃)CH₂CH₂CH₃ |
| A-128 | CH₂CH₂CH(CH₃)CH₂CH₂CH₂CH₂CH(CH₃)CH₂CH₃ |
| A-129 | CH₂CH₂CH₂CH(CH₃)CH₂CH₂CH₂CH(CH₃)CH₂CH₃ |
| A-130 | CH₂CH(CH₃)CH₂CH₂CH₂CH₂CH₂C(CH₃)₃ |
| A-131 | CH₂CH₂CH₂-O-CH₃ |
| A-132 | CH₂CH₂CH₂-O-CH₂CH₃ |
| A-133 | CH₂CH₂CH₂-O-CH₂CH₂CH₃ |
| A-134 | CH₂CH₂CH₂-O-CH₂CH₂CH₂CH₃ |
| A-135 | CH₂CH₂CH₂-O-CH₂CH₂CH₂CH₂CH₃ |
| A-136 | CH₂CH₂CH₂-O-CH₂CH₂CH₂CH₂CH₂CH₃ |
| A-137 | CH₂CH₂CH₂-O-CH₂CH₂CH₂CH₂CH₂CH₂CH₃ |
| A-138 | CH₂CH₂CH₂-O-CH₂CH₂CH₂CH₂CH₂CH₂CH₂CH₃ |
| A-139 | CH₂CH₂CH₂-O-CH₂CH₂CH₂CH₂CH₂CH₂CH₂CH₂CH₃ |
| A-140 | CH₂CH₂CH₂-O-CH(CH₃)₂ |
| A-141 | CH₂CH₂CH₂-O-C(CH₃)₃ |
| A-142 | CH₂CH₂CH₂-O-CH₂C(CH₃)₃ |
| A-143 | CH₂CH₂CH₂-O-CH(CH₃)CH₂C(CH₃)₃ |
| A-144 | CH₂CH₂CH₂-O-CH(CH₂CH₃)CH₂C(CH₃)₃ |
| A-145 | CH₂CH₂CH₂-O-CH₂CH(CH₃)CH₂CH(CH₃)₂ |
| A-146 | CH₂CH₂CH₂-O-CH₂CH(CH₂CH₃)CH₂CH₂CH₃ |
| A-147 | CH₂CH₂CH₂-O-CH₂CH₂CH(CH₃)CH₂CH(CH₃)₂ |
| A-148 | CH₂CH₂CH₂-O-CH₂CH₂CH(CH₃)CH₂C(CH₃)₃ |
| A-149 | CH₂CH₂CH₂-O-CH₂CH₂CH(CH₃)CH₂CH₂CH(CH₃)₂ |
| A-150 | CH₂CH₂CH₂-O-CH₂CH₂CH(CH₃)CH₂CH₂CH₂CH(CH₃)₂ |
| A-151 | CH₂CH₂CH₂CH₂-O-CH₃ |
| A-152 | CH₂CH₂CH₂CH₂-O-CH₂CH₃ |
| A-153 | CH₂CH₂CH₂CH₂-O-CH₂CH₂CH₃ |
| A-154 | CH₂CH₂CH₂CH₂-O-CH₂CH₂CH₂CH₃ |
| A-155 | CH₂CH₂CH₂CH₂-O-CH₂CH₂CH₂CH₂CH₃ |
| A-156 | CH₂CH₂CH₂CH₂-O-CH₂CH₂CH₂CH₂CH₂CH₃ |
| A-157 | CH₂CH₂CH₂CH₂-O-CH₂CH₂CH₂CH₂CH₂CH₂CH₃ |
| A-158 | CH₂CH₂CH₂CH₂-O-CH₂CH₂CH₂CH₂CH₂CH₂CH₂CH₃ |
| A-159 | CH₂CH₂CH₂CH₂-O-CH(CH₃)₂ |
| A-160 | CH₂CH₂CH₂CH₂-O-C(CH₃)₃ |
| A-161 | CH₂CH₂CH₂CH₂-O-CH₂C(CH₃)₃ |
| A-162 | CH₂CH₂CH₂CH₂-O-CH(CH₃)CH₂C(CH₃)₃ |
| A-163 | CH₂CH₂CH₂CH₂-O-CH(CH₂CH₃)CH₂C(CH₃)₃ |
| A-164 | CH₂CH₂CH₂CH₂-O-CH₂CH(CH₃)CH₂CH(CH₃)₂ |
| A-165 | CH₂CH₂CH₂CH₂-0-CH₂CH(CH₂CH₃)CH₂CH₂CH₃ |
| A-166 | CH₂CH₂CH₂CH₂-O-CH₂CH₂CH(CH₃)CH₂CH(CH₃)₂ |
| A-167 | CH₂CH₂CH₂CH₂-O-CH₂CH₂CH(CH₃)CH₂C(CH₃)₃ |
| A-168 | CH₂CH₂CH₂CH₂-O-CH₂CH₂CH(CH₃)CH₂CH₂CH(CH₃)₂ |
| A-169 | CH₂CH₂CH₂CH₂-O-CH₂CH₂CH(CH₃)CH₂CH₂CH₂CH(CH₃)₂ |
| A-170 | CH₂CH₂CH₂CH₂CH₂-O-CH₃ |
| A-171 | CH₂CH₂CH₂CH₂CH₂-O-CH₂CH₃ |
| A-172 | CH₂CH₂CH₂CH₂CH₂-O-CH₂CH₂CH₃ |
| A-173 | CH₂CH₂CH₂CH₂CH₂-O-CH₂CH₂CH₂CH₃ |
| A-174 | CH₂CH₂CH₂CH₂CH₂-O-CH₂CH₂CH₂CH₂CH₃ |
| A-175 | CH₂CH₂CH₂CH₂CH₂-O-CH₂CH₂CH₂CH₂CH₂CH₃ |
| A-176 | CH₂CH₂CH₂CH₂CH₂-O-CH₂CH₂CH₂CH₂CH₂CH₂CH₃ |
| A-177 | CH₂CH₂CH₂CH₂CH₂-O-CH₂CH₂CH₂CH₂CH₂CH₂CH₂CH₃ |
| A-178 | CH₂CH₂CH₂CH₂CH₂-O-CH(CH₃)₂ |
| A-179 | CH₂CH₂CH₂CH₂CH₂-O-C(CH₃)₃ |
| A-180 | CH₂CH₂CH₂CH₂CH₂-O-CH₂C(CH₃)₃ |
| A-181 | CH₂CH₂CH₂CH₂CH₂-O-CH(CH₃)CH₂C(CH₃)₃ |
| A-182 | CH₂CH₂CH₂CH₂CH₂-O-CH(CH₂CH₃)CH₂C(CH₃)₃ |
| A-183 | CH₂CH₂CH₂CH₂CH₂-O-CH₂CH(CH₃)CH₂CH(CH₃)₂ |
| A-184 | CH₂CH₂CH₂CH₂CH₂-O-CH₂CH(CH₂CH₃)CH₂CH₂CH₃ |
| A-185 | CH₂CH₂CH₂CH₂CH₂-O-CH₂CH₂CH(CH₃)CH₂CH₂CH(CH₃)₂ |
| A-186 | CH₂CH₂CH₂CH₂CH₂-O-CH₂CH₂CH(CH₃)CH₂CH(CH₃)₂ |
| A-187 | CH₂CH₂CH₂CH₂CH₂-O-CH₂CH₂CH(CH₃)CH₂C(CH₃)₃ |
| A-188 | Cyclopropyl |
| A-189 | Cyclopentyl |
| A-190 | Cyclohexyl |
| A-191 | CH=CH₂ |
| A-192 | CH₂CH=CH₂ |
| A-193 | CH=CHCH₃ |
| A-194 | C(CH₃)=CH₂ |
| A-195 | CH₂CH₂CH=CH₂ |
| A-196 | CH₂CH=CHCH₃ |
| A-197 | CH=CHCH₂CH₃ |
| A-198 | CH(CH₃)CH=CH₂ |
| A-199 | C(CH₃)=CHCH₃ |
| A-200 | CH=C(CH₃)₂ |
| A-201 | CH₂CH₂CH₂CH=CH₂ |
| A-202 | CH₂CH₂CH=CHCH₃ |
| A-203 | CH₂CH=CHCH₂CH₃ |
| A-204 | CH=CHCH₂CH₂CH₃ |
| A-205 | CH(CH₃)CH₂CH=CH₂ |
| A-206 | CH₂C(CH₃)=CHCH₃ |
| A-207 | CH₂CH=C(CH₃)₂ |
| A-208 | CH₂CH₂CH₂CH₂CH=CH₂ |
| A-209 | CH₂CH₂CH₂CH=CHCH₃ |
| A-210 | CH₂CH₂CH=CHCH₂CH₃ |
| A-211 | CH₂CH=CHCH₂CH₂CH₃ |
| A-212 | CH=CHCH₂CH₂CH₂CH₃ |
| A-213 | CH(CH₃)CH₂CH₂CH=CH₂ |
| A-214 | CH(CH₃)CH₂CH=CHCH₃ |
| A-215 | CH₂C(CH₃)=CHCH₂CH₃ |
| A-216 | CH₂CH₂CH=C(CH₃)₂ |
| A-217 | CH₂CH₂CH₂CH₂CH₂CH=CH₂ |
| A-218 | CH₂CH₂CH₂CH₂CH=CHCH₃ |
| A-219 | CH₂CH₂CH₂CH=CHCH₂CH₃ |
| A-220 | CH₂CH₂CH=CHCH₂CH₂CH₃ |
| A-221 | CH₂CH=CHCH₂CH₂CH₂CH₃ |
| A-222 | CH=CHCH₂CH₂CH₂CH₂CH₃ |
| A-223 | CH(CH₃)CH₂CH₂CH₂CH=CH₂ |
| A-224 | CH(CH₃)CH₂CH₂CH=CHCH₃ |
| A-225 | C(CH₃)=CHCH₂CH₂CH₂CH₃ |
| A-226 | CH₂CH₂CH₂CH=C(CH₃)₂ |
| A-227 | CH₂CH₂CH₂CH₂CH₂CH₂CH=CH₂ |
| A-228 | CH₂CH₂CH₂CH₂CH₂CH=CHCH₃ |
| A-229 | CH₂CH₂CH₂CH₂CH=CHCH₂CH₃ |
| A-230 | CH₂CH₂CH₂CH=CHCH₂CH₂CH₃ |
| A-231 | CH₂CH₂CH=CHCH₂CH₂CH₂CH₃ |
| A-232 | CH₂CH=CHCH₂CH₂CH₂CH₂CH₃ |
| A-233 | CH=CHCH₂CH₂CH₂CH₂CH₂CH₃ |
| A-234 | CH(CH₃)CH₂CH₂CH₂CH₂CH=CH₂ |
| A-235 | CH(CH₃)CH₂CH₂CH₂CH=CHCH₃ |
| A-236 | C(CH₃)=CHCH₂CH₂CH₂CH₂CH₃ |
| A-237 | CH₂CH₂CH₂CH₂CH=C(CH₃)₂ |
| A-238 | CH₂CH₂CH₂CH₂CH₂CH₂CH₂CH=CH₂ |
| A-239 | CH₂CH₂CH₂CH₂CH₂CH₂CH=CHCH₃ |
| A-240 | CH₂CH₂CH₂CH₂CH₂CH=CHCH₂CH₃ |
| A-241 | CH₂CH₂CH₂CH₂CH=CHCH₂CH₂CH₃ |
| A-242 | CH₂CH₂CH₂CH=CHCH₂CH₂CH₂CH₃ |
| A-243 | CH₂CH₂CH=CHCH₂CH₂CH₂CH₂CH₃ |
| A-244 | CH₂CH=CHCH₂CH₂CH₂CH₂CH₂CH₃ |
| A-245 | CH=CHCH₂CH₂CH₂CH₂CH₂CH₂CH₃ |
| A-246 | CH(CH₃)CH₂CH₂CH₂CH₂CH₂CH=CH₂ |
| A-247 | CH(CH₃)CH₂CH₂CH₂CH₂CH=CHCH₃ |
| A-248 | C(CH₃)=CHCH₂CH₂CH₂CH₂CH₂CH₃ |
| A-249 | CH₂CH₂CH₂CH₂CH₂CH=C(CH₃)₂ |
| A-250 | CH₂CH₂CH₂CH₂CH₂CH₂CH₂CH₂CH=CH₂ |
| A-251 | CH₂CH₂CH₂CH₂CH₂CH₂CH₂CH=CHCH₃ |
| A-252 | CH₂CH₂CH₂CH₂CH₂CH₂CH=CHCH₂CH₃ |
| A-253 | CH₂CH₂CH₂CH₂CH₂CH=CHCH₂CH₂CH₃ |
| A-254 | CH₂CH₂CH₂CH₂CH=CHCH₂CH₂CH₂CH₃ |
| A-255 | CH₂CH₂CH₂CH=CHCH₂CH₂CH₂CH₂CH₃ |
| A-256 | CH₂CH₂CH=CHCH₂CH₂CH₂CH₂CH₂CH₃ |
| A-257 | CH₂CH=CHCH₂CH₂CH₂CH₂CH₂CH₂CH₃ |
| A-258 | CH=CHCH₂CH₂CH₂CH₂CH₂CH₂CH₂CH₃ |
| A-259 | CH(CH₃)CH₂CH₂CH₂CH₂CH₂CH₂CH=CH₂ |
| A-260 | CH(CH₃)CH₂CH₂CH₂CH₂CH₂CH=CHCH₃ |
| A-261 | C(CH₃)=CHCH₂CH₂CH₂CH₂CH₂CH₂CH₃ |
| A-262 | CH₂CH₂CH₂CH₂CH₂CH₂CH=C(CH₃)₂ |
| A-263 | C≡CH |
| A-264 | CH₂C≡CH |
| A-265 | C≡CCH₃ |
| A-266 | CH₂CH₂C≡CH |
| A-267 | CH₂C≡CCH₃ |
| A-268 | C≡CCH₂CH₃ |
| A-269 | CH(CH₃)C≡CH |
| A-270 | CH₂CH₂CH₂C≡CH |
| A-271 | CH₂CH₂C≡CCH₃ |
| A-272 | CH₂C≡CCH₂CH₃ |
| A-273 | C≡CCH₂CH₂CH₃ |
| A-274 | CH(CH₃)CH₂C≡CH |
| A-275 | CH₂CH₂CH₂CH₂C≡CH |
| A-276 | CH₂CH₂CH₂C≡CCH₃ |
| A-277 | CH₂CH₂C≡CCH₂CH₃ |
| A-278 | CH₂C≡CCH₂CH₂CH₃ |
| A-279 | C≡CCH₂CH₂CH₂CH₃ |
| A-280 | CH(CH₃)CH₂CH₂C≡CH |
| A-281 | CH(CH₃)CH₂C≡CCH₃ |
| A-282 | CH₂CH₂CH₂CH₂CH₂C≡CH |
| A-283 | CH₂CH₂CH₂CH₂C≡CCH₃ |
| A-284 | CH₂CH₂CH₂C≡CCH₂CH₃ |
| A-285 | CH₂CH₂C≡CCH₂CH₂CH₃ |
| A-286 | CH₂C≡CCH₂CH₂CH₂CH₃ |
| A-287 | C≡CCH₂CH₂CH₂CH₂CH₃ |
| A-288 | CH(CH₃)CH₂CH₂CH₂C≡CH |
| A-289 | CH(CH₃)CH₂CH₂C≡CCH₃ |
| A-290 | CH(CH₃)CH₂C≡CCH₂CH₃ |
| A-291 | CH₂CH₂CH₂CH₂CH₂CH₂C≡CH |
| A-292 | CH₂CH₂CH₂CH₂CH₂C≡CCH₃ |
| A-293 | CH₂CH₂CH₂CH₂C≡CCH₂CH₃ |
| A-294 | CH₂CH₂CH₂C≡CCH₂CH₂CH₃ |
| A-295 | CH₂CH₂C≡CCH₂CH₂CH₂CH₃ |
| A-296 | CH₂C≡CCH₂CH₂CH₂CH₂CH₃ |
| A-297 | C≡CCH₂CH₂CH₂CH₂CH₂CH₃ |
| A-298 | CH(CH₃)CH₂CH₂CH₂CH₂C≡CH |
| A-299 | CH(CH₃)CH₂CH₂CH₂C≡CCH₃ |
| A-300 | CH₂CH₂CH₂CH₂CH₂CH₂CH₂C≡CH |
| A-301 | CH₂CH₂CH₂CH₂CH₂CH₂C≡CCH₃ |
| A-302 | CH₂CH₂CH₂CH₂CH₂C≡CCH₂CH₃ |
| A-303 | CH₂CH₂CH₂CH₂C≡CCH₂CH₂CH₃ |
| A-304 | CH₂CH₂CH₂C≡CCH₂CH₂CH₂CH₃ |
| A-305 | CH₂CH₂C≡CCH₂CH₂CH₂CH₂CH₃ |
| A-306 | CH₂C≡CCH₂CH₂CH₂CH₂CH₂CH₃ |
| A-307 | C≡CCH₂CH₂CH₂CH₂CH₂CH₂CH₃ |
| A-308 | CH(CH₃)CH₂CH₂CH₂CH₂CH₂C≡CH |
| A-309 | CH(CH₃)CH₂CH₂CH₂CH₂C≡CCH₃ |
| A-310 | CH₂CH₂CH₂CH₂CH₂CH₂CH₂CH₂C≡CH |
| A-311 | CH₂CH₂CH₂CH₂CH₂CH₂CH₂C≡CCH₃ |
| A-312 | CH₂CH₂CH₂CH₂CH₂CH₂C≡CCH₂CH₃ |
| A-313 | CH₂CH₂CH₂CH₂CH₂C≡CCH₂CH₂CH₃ |
| A-314 | CH₂CH₂CH₂CH₂C≡CCH₂CH₂CH₂CH₃ |
| A-315 | CH₂CH₂CH₂C≡CCH₂CH₂CH₂CH₂CH₃ |
| A-316 | CH₂CH₂C≡CCH₂CH₂CH₂CH₂CH₂CH₃ |
| A-317 | CH₂C≡CCH₂CH₂CH₂CH₂CH₂CH₂CH₃ |
| A-318 | C≡CCH₂CH₂CH₂CH₂CH₂CH₂CH₂CH₃ |
| A-319 | CH(CH₃)CH₂CH₂CH₂CH₂CH₂CH₂C≡CH |
| A-320 | CH(CH₃)CH₂CH₂CH₂CH₂CH₂C≡CCH₃ |
| A-321 | CH₂CH₂CN |
| A-322 | CH₂CH₂CH₂CN |
| A-323 | CH₂CH₂CH₂CH₂CN |
| A-324 | CH₂CH₂CH₂CH₂CH₂CN |
| A-325 | CH₂CH₂CH₂CH₂CH₂CH₂CN |
| A-326 | CH₂CH₂CH₂CH₂CH₂CH₂CH₂CN |
| A-327 | CH₂CH₂CH₂CH₂CH₂CH₂CH₂CH₂CN |
| A-328 | CH₂CH₂CH₂CH₂CH₂CH₂CH₂CH₂CH₂CN |
| A-329 | CH₂CH₂CH₂CH₂CH₂CH₂CH₂CH₂CH₂CH₂CN |

Die Verbindungen I eignen sich als Fungizide. Sie zeichnen sich aus durch eine hervorragende Wirksamkeit gegen ein breites Spektrum von pflanzenpathogenen Pilzen aus der Klasse der *Ascomyceten, Deuteromyceten, Oomyceten* und *Basidiomyceten,* insbesondere aus der Klasse der *Oomyceten.* Sie sind zum Teil systemisch wirksam und können im Pflanzenschutz als Blatt-, Beiz- und Bodenfungizide eingesetzt werden.

Besondere Bedeutung haben sie für die Bekämpfung einer Vielzahl von Pilzen an verschiedenen Kulturpflanzen wie Weizen, Roggen, Gerste, Hafer, Reis, Mais, Gras, Bananen, Baumwolle, Soja, Kaffee, Zuckerrohr, Wein, Obst- und Zierpflanzen und Gemüsepflanzen wie Gurken, Bohnen, Tomaten, Kartoffeln und Kürbissen, sowie an den Samen dieser Pflanzen.

Speziell eignen sie sich zur Bekämpfung folgender Pflanzenkrankheiten:
- *Alternaria* Arten an Gemüse, Raps, Zuckerrüben und Obst und Reis,
- *Aphanomyces* Arten an Zuckerrüben und Gemüse,
- *Bipolaris-* und *Drechslera* Arten an Mais, Getreide, Reis und Rasen,
- *Blumeria graminis* (Echter Mehltau) an Getreide,
- *Botrytis cinerea* (Grauschimmel) an Erdbeeren, Gemüse, Blumen und Weinreben,
- *Bremia lactucae* an Salat,
- *Cercospora* Arten an Mais, Sojabohnen, Reis und Zuckerrüben,
- *Cochliobolus* Arten an Mais, Getreide, Reis (z.B. *Cochliobolus sativus* an Getreide, *Cochliobolus miyabeanus* an Reis),
- *Colletotricum* Arten an Sojabohnen und Baumwolle,
- *Drechslera Arten* an Getreide und Mais,
- *Exserohilum* Arten an Mais,
- *Erysiphe cichoracearum* und *Sphaerotheca fuliginea* an Gurkengewächsen,
- *Fusarium* und *Verticillium* Arten an verschiedenen Pflanzen
- *Gaeumanomyces graminis* an Getreide
- *Gibberella* Arten an Getreide und Reis (z.B. *Gibberella fujikuroi* an Reis)
- *Grainstaining complex* an Reis,
- *Helminthosporium* Arten an Mais und Reis,
- *Michrodochium nivale* an Getreide,
- *Mycosphaerella* Arten an Getreide, Bananen und Erdnüssen,
- *Phakopsara pachyrhizi* und *Phakopsara meibomiae* an Sojabohnen,
- *Phomopsis* Arten an Sojabohnen und Sonnenblumen,
- *Phytophthora infestans* an Kartoffeln und Tomaten,
- *Plasmopara viticola* an Weinreben,
- *Podosphaera leucotricha* an Apfel,
- *Pseudocercosporella herpotrichoides* an Getreide,
- *Pseudoperonospora* Arten an Hopfen und Gurkengewächsen,
- *Puccinia* Arten an Getreide und Mais,
- *Pyrenophora* Arten an Getreide,
- *Pyricularia oryzae, Corticium sasakii, Sarocladium oryzae, S. attenuatum, Entyloma oryzae* an Reis,
- *Pyricularia grisea* an Rasen und Getreide,
- *Pythium spp.* an Rasen, Reis, Mais, Baumwolle, Raps, Sonnenblumen, Zuckerrüben, Gemüse und anderen Pflanzen,
- *Rhizoctonia-Arten* an Baumwolle, Reis, Kartoffeln, Rasen, Mais, Raps, Kartoffeln, Zuckerrüben, Gemüse und anderen Pflanzen,
- *Sclerotinia* Arten an Raps und Sonnenblumen,
- *Septoria tritici* und *Stagonospora nodorum* an Weizen,
- *Erysiphe* (syn. *Uncinula*) *necator* an Weinrebe,
- *Setospaeria* Arten an Mais und Rasen,
- *Sphacelotheca reilinia* an Mais,
- *Thievaliopsis* Arten an Sojabohnen und Baumwolle,
- *Tilletia Arten* an Getreide,
- *Ustilago* Arten an Getreide, Mais und Zuckerrübe und
- *Venturia* Arten (Schorf) an Apfel und Birne.

Insbesondere eignen sie sich zur Bekämpfung von Schadpilzen aus der Klasse der *Oomyceten,* wie Peronospora-Arten, *Phytophthora-Arten, Plasmopara viticola* und *Pseudoperonospora-Arten.*

Die Verbindungen I eignen sich außerdem zur Bekämpfung von Schadpilzen im Materialschutz (z.B. Holz, Papier, Dispersionen für den Anstrich, Fasern bzw. Gewebe) und im Vorratsschutz. Im Holzschutz finden insbesondere folgende Schadpilze Beachtung: Ascomyceten wie *Ophiostoma* spp., *Ceratocystis* spp., *Aureobasidium pullulans, Sclerophoma* spp., *Chaetomium* spp*., Humicola* spp., *Petriella* spp., *Trichurus* spp.; Basidiomyceten wie *Coniophora* spp., *Coriolus* spp., *Gloeophyllum* spp., *Lentinus* spp., *Pleurotus* spp., *Poria* spp., *Serpula* spp. und *Tyromyces* spp., Deuteromyceten wie *Aspergillus* spp., *Cladosporium* spp., *Penicillium* spp., *Trichoderma* spp., *Alternaria* spp., *Paecilomyces* spp. und Zygomyceten wie *Mucor* spp.*,* darüber hinaus im Materialschutz folgende Hefepilze: *Candida* spp. und *Saccharomyces cerevisae*.

Die Verbindungen I werden angewendet, indem man die Pilze oder die vor Pilzbefall zu schützenden Pflanzen, Saatgüter, Materialien oder den Erdboden mit einer fungizid wirksamen Menge der Wirkstoffe behandelt. Die Anwendung kann sowohl vor als auch nach der Infektion der Materialien, Pflanzen oder Samen durch die Pilze erfolgen.

Die fungiziden Mittel enthalten im allgemeinen zwischen 0,1 und 95, vorzugsweise zwischen 0,5 und 90 Gew.-% Wirkstoff.

Die Aufwandmengen liegen bei der Anwendung im Pflanzenschutz je nach Art des gewünschten Effektes zwischen 0,01 und 2,0 kg Wirkstoff pro ha.

Bei der Saatgutbehandlung werden im allgemeinen Wirkstoffmengen von 1 bis 1000 g/100 kg, vorzugsweise 5 bis 100 g/100 kg Saatgut benötigt.

Bei der Anwendung im Material- bzw. Vorratsschutz richtet sich die Aufwandmenge an Wirkstoff nach der Art des Einsatzgebietes und des gewünschten Effekts. Übliche Aufwandmengen sind im Materialschutz beispielsweise 0,001 g bis 2 kg, vorzugsweise 0,005 g bis 1 kg Wirkstoff pro Kubikmeter behandelten Materials.

Die Verbindungen der Formel I können in verschiedenen Kristallmodifikationen vorliegen, die sich in der biologischen Wirksamkeit unterscheiden können. Sie sind ebenfalls Gegenstand der vorliegenden Erfindung.

Die Verbindungen I können in die üblichen Formulierungen überführt werden, z.B. Lösungen, Emulsionen, Suspensionen, Stäube, Pulver, Pasten und Granulate. Die Anwendungsform richtet sich nach dem jeweiligen Verwendungszweck; sie soll in jedem Fall eine feine und gleichmäßige Verteilung der erfindungsgemäßen Verbindung gewährleisten.

Die Formulierungen werden in bekannter Weise hergestellt, z.B. durch Verstrecken des Wirkstoffs mit Lösungsmitteln und/oder Trägerstoffen, gewünschtenfalls unter Verwendung von Emulgiermitteln und Dispergiermitteln. Als Lösungsmittel / Hilfsstoffe kommen dafür im wesentlichen in Betracht:
- Wasser, aromatische Lösungsmittel (z.B. Solvesso Produkte, Xylol), Paraffine (z.B. Erdölfraktionen), Alkohole (z.B. Methanol, Butanol, Pentanol, Benzylalkohol), Ketone (z.B. Cyclohexanon, gamma-Butryolacton), Pyrrolidone (NMP, NOP), Acetate (Glykoldiacetat), Glykole, Dimethylfettsäureamide, Fettsäuren und Fettsäureester. Grundsätzlich können auch Lösungsmittelgemische verwendet werden,
- Trägerstoffe wie natürliche Gesteinsmehle (z.B. Kaoline, Tonerden, Talkum, Kreide) und synthetische Gesteinsmehle (z.B. hochdisperse Kieselsäure, Silikate); Emulgiermittel wie nichtionogene und anionische Emulgatoren (z.B. Polyoxyethylen-Fettalkohol-Ether, Alkylsulfonate und Arylsulfonate) und Dispergiermittel wie LigninSulfitablaugen und Methylcellulose.

Als oberflächenaktive Stoffe kommen Alkali-, Erdalkali-, Ammoniumsalze von Ligninsulfonsäure, Naphthalinsulfonsäure, Phenolsulfonsäure, Dibutylnaphthalinsulfonsäure, Alkylarylsulfonate, Alkylsulfate, Alkylsulfonate, Fettalkoholsulfate, Fettsäuren und sulfatierte Fettalkoholglykolether zum Einsatz, ferner Kondensationsprodukte von sulfoniertem Naphthalin und Naphthalinderivaten mit Formaldehyd, Kondensationsprodukte des Naphthalins bzw. der Naphtalinsulfonsäure mit Phenol und Formaldehyd, Polyoxyethylenoctylphenolether, ethoxyliertes Isooctylphenol, Octylphenol, Nonylphenol, Alkylphenolpolyglykolether, Tributylphenylpolyglykolether, Tristerylphenylpolyglykolether, Alkylarylpolyetheralkohole, Alkohol- und Fettalkoholethylenoxid-Kondensate, ethoxyliertes Rizinusöl, Polyoxyethylenalkylether, ethoxyliertes Polyoxypropylen, Laurylalkoholpolyglykoletheracetal, Sorbitester, Ligninsulfitablaugen und Methylcellulose in Betracht.

Zur Herstellung von direkt versprühbaren Lösungen, Emulsionen, Pasten oder Öldispersionen kommen Mineralölfraktionen von mittlerem bis hohem Siedepunkt, wie Kerosin oder Dieselöl, ferner Kohlenteeröle sowie Öle pflanzlichen oder tierischen Ursprungs, aliphatische, cyclische und aromatische Kohlenwasserstoffe, z.B. Toluol, Xylol, Paraffin, Tetrahydronaphthalin, alkylierte Naphthaline oder deren Derivate, Methanol, Ethanol, Propanol, Butanol, Cyclohexanol, Cyclohexanon, Isophoron, stark polare Lösungsmittel, z.B. Dimethylsulfoxid, N-Methylpyrrolidon oder Wasser in Betracht.

Pulver-, Streu- und Stäubemittel können durch Mischen oder gemeinsames Vermahlen der wirksamen Substanzen mit einem festen Trägerstoff hergestellt werden.

Granulate, z.B. Umhüllungs-, Imprägnierungs- und Homogengranulate, können durch Bindung der Wirkstoffe an feste Trägerstoffe hergestellt werden. Feste Trägerstoffe sind z.B. Mineralerden, wie Kieselgele, Silikate, Talkum, Kaolin, Attaclay, Kalkstein, Kalk, Kreide, Bolus, Löß, Ton, Dolomit, Diatomeenerde, Calcium- und Magnesiumsulfat, Magnesiumoxid, gemahlene Kunststoffe, Düngemittel, wie z.B. Ammoniumsulfat, Ammoniumphosphat, Ammoniumnitrat, Harnstoffe und pflanzliche Produkte, wie Getreidemehl, Baumrinden-, Holz- und Nußschalenmehl, Cellulosepulver und andere feste Trägerstoffe.

Die Formulierungen enthalten im allgemeinen zwischen 0,01 und 95 Gew.-%, vorzugsweise zwischen 0,1 und 90 Gew.-% des Wirkstoffs. Die Wirkstoffe werden dabei in einer Reinheit von 90% bis 100%, vorzugsweise 95% bis 100% (nach NMR-Spektrum) eingesetzt.

### Beispiele für Formulierungen sind: 1. Produkte zur Verdünnung in Wasser

### A Wasserlösliche Konzentrate (SL, LS)

10 Gew.-Teile der Wirkstoffe werden mit 90 Gew.-Teilen Wasser oder einem wasserlöslichen Lösungsmittel gelöst. Alternativ werden Netzmittel oder andere Hilfsmittel zugefügt. Bei der Verdünnung in Wasser löst sich der Wirkstoff. Man erhält auf diese Weise eine Formulierung mit 10 Gew.-% Wirkstoffgehalt.

### B Dispergierbare Konzentrate (DC)

20 Gew.-Teile der Wirkstoffe werden in 70 Gew.-Teilen Cyclohexanon unter Zusatz von 10 Gew.-Teilen eines Dispergiermittels z.B. Polyvinylpyrrolidon gelöst. Bei Verdünnung in Wasser ergibt sich eine Dispersion. Der Wirkstoffgehalt beträgt 20 Gew.-%

### C Emulgierbare Konzentrate (EC)

15 Gew.-Teile der Wirkstoffe werden in 75 Gew.-Teilen Xylol unter Zusatz von Ca-Dodecylbenzolsulfonat und Ricinusölethoxylat (jeweils 5 Gew.-Teile) gelöst. Bei der Verdünnung in Wasser ergibt sich eine Emulsion. Die Formulierung hat 15 Gew.-% Wirkstoffgehalt.

### D Emulsionen (EW, EO, ES)

25 Gew.-Teile der Wirkstoffe werden in 35 Gew.-Teile Xylol unter Zusatz von Ca-Dodecylbenzolsulfonat und Ricinusölethoxylat (jeweils 5 Gew.-Teile) gelöst. Diese Mischung wird mittels einer Emulgiermaschine (z.B. Ultraturax) in 30 Gew.Teile Wasser gegeben und zu einer homogenen Emulsion gebracht. Bei der Verdünnung in Wasser ergibt sich eine Emulsion. Die Formulierung hat einen Wirkstoffgehalt von 25 Gew.-%.

### E Suspensionen (SC, OD, FS)

20 Gew.-Teile der Wirkstoffe werden unter Zusatz von 10 Gew.-Teilen Dispergier- und Netzmitteln und 70 Gew.-Teilen Wasser oder einem organischen Lösungsmittel in einer Rührwerkskugelmühle zu einer feinen Wirkstoffsuspension zerkleinert. Bei der Verdünnung in Wasser ergibt sich eine stabile Suspension des Wirkstoffs. Der Wirkstoffgehalt in der Formulierung beträgt 20 Gew.-% .

### F Wasserdispergierbare und wasserlösliche Granulate (WG, SG)

50 Gew.-Teile der Wirkstoffe werden unter Zusatz von 50 Gew-Teilen Dispergier- und Netzmitteln fein gemahlen und mittels technischer Geräte (z.B. Extrusion, Sprühturm, Wirbelschicht) als wasserdispergierbare oder-wasserlösliche-Granulate hergestellt. Bei der Verdünnung in Wasser ergibt sich eine stabile Dispersion oder Lösung des Wirkstoffs. Die Formulierung hat einen Wirkstoffgehalt von 50 Gew.-%.

### G Wasserdispergierbare und wasserlösliche Pulver (WP, SP, SS, WS)

75 Gew.-Teile der Wirkstoffe werden unter Zusatz von 25 Gew.-Teilen Dispergier- und Netzmitteln sowie Kieselsäuregel in einer Rotor-Strator Mühle vermahlen. Bei der Verdünnung in Wasser ergibt sich eine stabile Dispersion oder Lösung des Wirkstoffs. Der Wirkstoffgehalt der Formulierung beträgt 75 Gew.-%.

### H Gelformulierungen

In einer Kugelmühle werden 20 Gew.-Teile der Wirkstoffe, 10 Gew.-Teile Dispergiermittel, 1Gew.-Teil Geliermittel und 70 Gew.-Teile Wasser oder eines organischen Lösungsmittels zu einer feinen Suspension vermahlen. Bei der Verdünnung mit Wasser ergibt sich eine stabile Suspension mit 20 Gew.-% Wirkstoffgehalt.

### 2. Produkte für die Direktapplikation

### I Stäube (DP, DS)

5 Gew.-Teile der Wirkstoffe werden fein gemahlen und mit 95 Gew.-Teilen feinteiligem Kaolin innig vermischt. Man erhält dadurch ein Stäubemittel mit 5 Gew.-% Wirkstoffgehalt.

### J Granulate (GR, FG, GG, MG)

0,5 Gew-Teile der Wirkstoffe werden fein gemahlen und mit 99,5 Gewichtsteilen Trägerstoffe verbunden. Gängige Verfahren sind dabei die Extrusion, die Sprühtrocknung oder die Wirbelschicht. Man erhält dadurch ein Granulat für die Direktapplikation mit 0,5 Gew.-% Wirkstoffgehalt.

### K ULV- Lösungen (UL)

10 Gew.-Teile der Wirkstoffe werden in 90 Gew.-Teilen eines organischen Lösungsmittel z.B. Xylol gelöst. Dadurch erhält man ein Produkt für die Direktapplikation mit 10 Gew.-% Wirkstoffgehalt.

Für die Saatgutbehandlung werden üblicherweise wasserlösliche Konzentrate (LS), Suspensionen (FS), Stäube (DS), wasserdispergierbare und wasserlösliche Pulver (WS, SS), Emulsionen (ES), emulgierbare Konzentrate (EC) und Gelformulierungen (GF) verwendet. Diese Formulierungen können auf das Saatgut unverdünnt oder, bevorzugt, verdünnt angewendet werden. Die Anwendung kann vor der Aussaat erfolgen.

Die Wirkstoffe können als solche, in Form ihrer Formulierungen oder den daraus bereiteten Anwendungsformen, z.B. in Form von direkt versprühbaren Lösungen, Pulvern, Suspensionen oder Dispersionen, Emulsionen, Öldispersionen, Pasten, Stäubemitteln, Streumitteln, Granulaten durch Versprühen, Vernebeln, Verstäuben, Verstreuen oder Gießen angewendet werden. Die Anwendungsformen richten sich ganz nach den Verwendungszwecken; sie sollten in jedem Fall möglichst die feinste Verteilung der erfindungsgemäßen Wirkstoffe gewährleisten.

Wässrige Anwendungsformen können aus Emulsionskonzentraten, Pasten oder netzbaren Pulvern (Spritzpulver, Öldispersionen) durch Zusatz von Wasser bereitet werden. Zur Herstellung von Emulsionen, Pasten oder Öldispersionen können die Substanzen als solche oder in einem Öl oder Lösungsmittel gelöst, mittels Netz-, Haft-, Dispergier- oder Emulgiermitttel in Wasser homogenisiert werden. Es können aber auch aus wirksamer Substanz Netz-, Haft-, Dispergier- oder Emulgiermittel und eventuell Lösungsmittel oder Öl bestehende Konzentrate hergestellt werden, die zur Verdünnung mit Wasser geeignet sind.

Die Wirkstoffkonzentrationen in den anwendungsfertigen Zubereitungen können in größeren Bereichen variiert werden. Im allgemeinen liegen sie zwischen 0,0001 und 10%, vorzugsweise zwischen 0,01 und 1%.

Die Wirkstoffe können auch mit gutem Erfolg im Ultra-Low-Volume-Verfahren (ULV) verwendet werden, wobei es möglich ist, Formulierungen mit mehr als 95 Gew.-% Wirkstoff oder sogar den Wirkstoff ohne Zusätze auszubringen.

Zu den Wirkstoffen können Öle verschiedenen Typs, Netzmittel, Adjuvants, Herbizide, Fungizide, andere Schädlingsbekämpfungsmittel, Bakterizide, gegebenenfalls auch erst unmittelbar vor der Anwendung (Tankmix), zugesetzt werden. Diese Mittel können zu den erfindungsgemäßen Mitteln im Gewichtsverhältnis 1:100 bis 100:1, bevorzugt 1:10 bis 10:1 1 zugemischt werden.

Als Adjuvants in diesem Sinne kommen insbesondere in Frage: organisch modifizierte Polysiloxane, z.B. Break Thru S 240^{®}; Alkoholalkoxylate, z. B. Atplus 245^{®}, Atplus MBA 1303^{®}, Plurafac LF 300^{®} und Lutensol ON 30^{®}; EO-PO-Blockpolymerisate, z. B. Pluronic RPE 2035^{®} und Genapol B^{®}; Alkoholethoxylate, z. B. Lutensol XP 80^{®}; und Natriumdioctylsulfosuccinat, z. B. Leophen RA^{®}.

Die erfindungsgemäßen Mittel können in der Anwendungsform als Fungizide auch zusammen mit anderen Wirkstoffen vorliegen, der z.B. mit Herbiziden, Insektiziden, Wachstumsregulatoren, Fungiziden oder auch mit Düngemitteln. Beim Vermischen der Verbindungen I bzw. der sie enthaltenden Mittel in der Anwendungsform als Fungizide mit anderen Fungiziden erhält man in vielen Fällen eine Vergrößerung des fungiziden Wirkungsspektrums.

Die folgende Liste von Fungiziden; mit denen die erfindungsgemäßen Verbindungen gemeinsam angewendet werden können, soll die Kombinationsmöglichkeiten erläutern, nicht aber einschränken:

### Strobilurine

Azoxystrobin, Dimoxystrobin, Enestroburin, Fluoxastrobin, Kresoxim-methyl, Metominostrobin, Picoxystrobin, Pyraclostrobin, Trifloxystrobin, Orysastrobin, (2-Chlor-5-[1-(3-methyl-benzyloxyimino)-ethyl]-benzyl)-carbaminsäuremethylester, (2-Chlor-5-[1-(6-methyl-pyridin-2-ylmethoxyimino)-ethyl]-benzyl)-carbaminsäuremethyl ester, 2-(ortho-(2,5-Dimethylphenyl-oxymethylen)phenyl)-3-methoxy-acrylsäuremethylester;

### Carbonsäureamide

- Carbonsäureanilide: Benalaxyl, Benodanil, Boscalid, Carboxin, Mepronil, Fenfuram, Fenhexamid, Flutolanil, Furametpyr, Metalaxyl, Ofurace, Oxadixyl, Oxycarboxin, Penthiopyrad, Thifluzamide, Tiadinil, 4-Difluormethyl-2-methyl-thiazol-5-carbonsäure-(4'-brom-biphenyl-2-yl)-amid, 4-Difluormethyl-2-methyl-thiazol-5-carbonsäure-(4'-trifluormethyl-biphenyl-2-yl)-amid, 4-Difluormethyl-2-methyl-thiazol-5-carbonsäure-(4'-chlor-3'-fluor-biphenyl-2-yl)-amid, 3-Difluormethyl-1-methyl-pyrazol-4-carbonsäure-(3',4'-dichlor-4-fluor-biphenyl-2-yl)-amid, 3,4-Dichlor-isothiazol-5-carbonsäure-(2-cyano-phenyl)-amid;
- Carbonsäuremorpholide: Dimethomorph, Flumorph;
- Benzoesäureamide: Flumetover, Fluopicolide (Picobenzamid), Zoxamide;
- Sonstige Carbonsäureamide: Carpropamid, Diclocymet, Mandipropamid, N-(2-(4-[3-(4-Chlor-phenyl)-prop-2-inyloxy]-3-methoxy-phenyl)-ethyl)-2-methansulfonylamino-3-methyl-butyramid, N-(2-(4-[3-(4-Chlor-phenyl)-prop-2-inyloxy]-3-methoxy-phenyl)-ethyl)-2-ethansulfonylamino-3-methyl-butyramid;

### Azole

- Triazole: Bitertanol, Bromuconazole, Cyproconazole, Difenoconazole, Diniconazole, Enilconazole, Epoxiconazole, Fenbuconazole, Flusilazole, Fluquinconazole, Flutriafol, Hexaconazol, Imibenconazole, Ipconazole, Metconazol, Myclobutanil, Penconazole, Propiconazole, Prothioconazole, Simeconazole, Tebuconazole, Tetraconazole, Triadimenol, Triadimefon, Triticonazole;
- Imidazole: Cyazofamid, Imazalil, Pefurazoate, Prochloraz, Triflumizole;
- Benzimidazole: Benomyl, Carbendazim, Fuberidazole, Thiabendazole;
- Sonstige: Ethaboxam, Etridiazole, Hymexazole;

### Stickstoffhaltige Heterocyclylverbindungen

- Pyridine: Fluazinam, Pyrifenox, 3-[5-(4-Chlor-phenyl)-2,3-dimethyl-isoxazolidin-3-yl]-pyridin;
- Pyrimidine: Bupirimate, Cyprodinil, Ferimzone, Fenarimol, Mepanipyrim, Nuarimol, Pyrimethanil;
- Piperazine: Triforine;
- Pyrrole: Fludioxonil, Fenpiclonil;
- Morpholine: Aldimorph, Dodemorph, Fenpropimorph, Tridemorph;
- Dicarboximide: Iprodione, Procymidone, Vinclozolin;
- sonstige: Acibenzolar-S-methyl, Anilazin, Captan, Captafol, Dazomet, Diclomezine, Fenoxanil, Folpet, Fenpropidin, Famoxadone, Fenamidone, Octhilinone, Probenazole, Proquinazid, Pyroquilon, Quinoxyfen, Tricyclazole, 5-Chlor-7-(4-methyl-piperidin-1-yl)-6-(2,4,6-trifluor-phenyl)-[1,2,4]triazolo[1,5-a]pyrimidin, 2-Butoxy-6-iodo-3-propyl-chromen-4-on, 3-(3-Brom-6-fluoro-2-methyl-indol-1-sulfonyl)-[1,2,4]triazol-1-sulfonsäuredimethylamid;

### Carbamate und Dithiocarbamate

- Dithiocarbamate: Ferbam, Mancozeb, Maneb, Metiram, Metam, Propineb, Thiram, Zineb, Ziram;
- Carbamate: Diethofencarb, Flubenthiavalicarb, Iprovalicarb, Propamocarb, 3-(4-Chlor-phenyl)-3-(2-isopropoxycarbonylamino-3-methyl-butyrylamino)-propionsäuremethylester, N-(1-(1-(4-cyanophenyl)ethansulfonyl)-but-2-yl) carbaminsäure-(4-fluorphenyl)ester;

### Sonstige Fungizide

- Guanidine: Dodine, Iminoctadine, Guazatine;
- Antibiotika: Kasugamycin, Polyoxine, Streptomycin, Validamycin A;
- Organometallverbindungen: Fentin Salze;
- Schwefelhaltige Heterocyclylverbindungen: Isoprothiolane, Dithianon;
- Organophosphorverbindungen: Edifenphos, Fosetyl, Fosetyl-aluminium, Iprobenfos, Pyrazophos, Tolclofos-methyl, Phosphorige Säure und ihre Salze;
- Organochlorverbindungen: Thiophanate Methyl, Chlorothalonil, Dichlofluanid, Tolylfluanid, Flusulfamide, Phthalide, Hexachlorbenzene, Pencycuron, Quintozene;
- Nitrophenylderivate: Binapacryl, Dinocap, Dinobuton;
- Anorganische Wirkstoffe: Bordeaux Brühe, Kupferacetat, Kupferhydroxid, Kupferoxychlorid, basisches Kupfersulfat, Schwefel;
- Sonstige: Spiroxamine, Cyflufenamid, Cymoxanil, Metrafenone.

### Synthesebeispiele

Die in den nachstehenden Synthesebeispielen wiedergegebenen Vorschriften wurden unter entsprechender Abwandlung der Ausgangsverbindungen zur Gewinnung weiterer Verbindungen I benutzt. Die so erhaltenen Verbindungen sind in der anschließenden Tabelle mit physikalischen Angaben aufgeführt.

### Beispiel 1: Herstellung von 3-Cyano-1-methoxyundecanon

Eine Suspension von 20,0 g (169 mmol) Kalium-tert-butylat in 120 ml wasserfr. Dimethylformamid (DMF) wurde mit 12,2 g (80 mmol) Decanitril und 11,0 g (106 mmol) Methoxyessigsäuremethylester versetzt. Nach 18 Std. Rühren bei 20-25°C wurde das Lösungsmittel abdestilliert, der Rückstand in Wasser aufgenommen und mit Cyclohexan gewaschen. Die wässrige Phase wurde mit konz. Salzsäure angesäuert und mit Diethylether extrahiert. Die vereinigten Etherphasen wurden mit Wasser gewaschen, getrocknet und vom Lösungsmittel befreit. Es bleiben 8,4 g der Titelverbindung als Öl zurück, die ohne weitere Reinigung weiter umgesetzt wurde.

### Beispiel 2: Herstellung von 7-Amino-5-methoxymethyl-6-octyl-triazolo(1,5-a)-pyrimidin

Eine Lösung von 22,0 g des Ketonitrils aus Bsp. 1, 8,1 g (97 mmol) 3-Amino-1,2,4-triazol und 3,8 g p-Toluolsulfonsäure in 60 ml Mesitylen wurde drei Std. auf 180°C erhitzt, wobei etwas Lösungsmittel abdestillierte. Dann wurde das Lösungsmittel vollständig abdestilliert und der Rückstand in Dichlormethan aufgenommen. Nach Waschen mit gesätt. NaHCO₃-Lsg. und Wasser wurde die organische Phase getrocknet, vom Lösungsmittel befreit und der Rückstand mit Diethylether digeriert. Es blieben 15,0 g der Titelverbindung als weiße Kristalle vom Fp. 180-181°C zurück.

**Tabelle I - Verbindungen der Formel I**

| Nr. | R¹ | R² | R³ | A | Phys. Daten (Fp. [°C]) |
|---|---|---|---|---|---|
| I-1 | (CH₂)₇CH₃ | CH₂OCH₃ | H | N | 180-181 |
| I-2 | (CH₂)₇CH₃ | CH₂OCH₂CH₃ | H | N | 180-181 |
| 1-3 | (CH₂)₃O(CH₂)₅CH₃ | CH₂OCH₃ | H | N | 133-134 |
| 1-4 | (CH₂)₃O(CH₂)₇CH₃ | CH₂OCH₃ | H | N | 127-128 |
| I-5 | (CH₂)₂CH(CH₃)CH₂C(CH₃)₃ | CH₂OCH₃ | H | N | 188-189 |
| I-6 | (CH₂)₉CH₃ | (CH₂)₃S(4-CH₃-C₆H₄) | H | N | 125-127 |

### Beispiele für die Wirkung gegen Schadpilze

Die fungizide Wirkung der Verbindungen der Formel I ließ sich durch die folgenden Versuche zeigen:

Die Wirkstoffe wurden als eine Stammlösung aufbereitet mit 25 mg Wirkstoff, welcher mit einem Gemisch aus Aceton und/oder DMSO und dem Emulgator Uniperol® EL (Netzmittel mit Emulgier- und Dispergierwirkung auf der Basis ethoxylierter Alkylphenole) im Volumen-Verhältnis Lösungsmittel-Emulgator von 99 zu 1 ad 10 ml aufgefüllt wurde. Anschließend wurde ad 100 ml mit Wasser aufgefüllt. Diese Stammlösung wurde mit dem beschriebenen Lösungsmittel-Emulgator-Wasser Gemisch zu der unten angegeben Wirkstoffkonzentration verdünnt.

Als Vergleichsverbindungen wurden die aus EP-A 141 317 als Beispiele Nr. 4, bzw. 42 bekannten Wirkstoffe A und B verwendet:

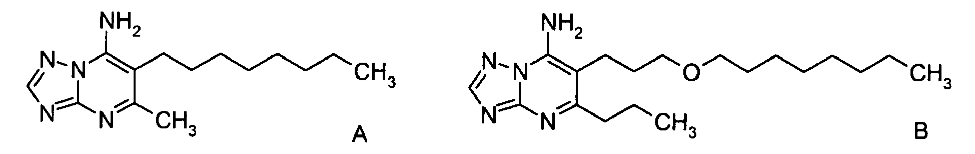

### Anwendungsbeispiel 1 - Aktivität gegen die Krautfäule an Tomaten verursacht durch Phytophthora infestans bei protektiver Behandlung

Blätter von getopften Tomatenpflanzen wurden mit einer wässriger Suspension in der unten angegebenen Wirkstoffkonzentration bis zur Tropfnässe besprüht. 1, bzw. 7 Tage nach der Applikation wurden die Blätter mit einer wässrigen Sporangienaufschwemmung von *Phytophthora infestans* infiziert. Anschließend wurden die Pflanzen in einer wasserdampf-gesättigten Kammer bei Temperaturen zwischen 18 und 20□C aufgestellt. Nach sechs Tagen hatte sich die Krautfäule auf den unbehandelten, jedoch infizierten Kontrollpflanzen so stark entwickelt, dass der Befall visuell in % ermittelt werden konnte.

In den Tests mit 1 Tag protektiver Behandlung zeigten die mit 16 ppm der Verbindung I-1 behandelten Pflanzen 15 % Befall, während die mit 16 ppm der Vergleichsverbindung A behandelten Pflanzen zu 70 % und die unbehandelten Pflanzen zu 90% befallen waren. In diesen Tests zeigten die mit 250 ppm der Verbindung I-4 behandelten Pflanzen nur 1 % Befall, während die mit 250 ppm der Vergleichsverbindung B behandelten und die unbehandelten Pflanzen zu 90 % befallen waren.

In einer weiteren Versuchsanstellung mit 1 Tag protektiver Behandlung zeigten die mit 63 ppm der Verbindungen I-1, I-4, bzw. I-5 behandelten Pflanzen maximal 5% Befall, während die unbehandelten Pflanzen zu 90% befallen waren.

In einer weiteren Versuchsanstellung mit 3 Tagen protektiver Behandlung zeigten die mit 250 ppm der Verbindungen I-1, bzw. I-4 behandelten Pflanzen maximal 20% Befall, während die mit 250 ppm der Vergleichsverbindungen A und B behandelten, wie auch die unbehandelten Pflanzen zu 90 % befallen waren.

In einer anderen Versuchsansstellung mit 7 Tagen protektiver Behandlung zeigten die mit 63 ppm der Verbindung I-1 behandelten Pflanzen maximal 5% Befall, während die unbehandelten Pflanzen zu 90 % befallen waren.

### Anwendungsbeispiel 2 - Dauer-Wirksamkeit gegen Rebenperonospora verursacht durch Plasmopara viticola bei protektiver Behandlung

Blätter von Topfreben wurden mit wässriger Suspension in der unten angegebenen Wirkstoffkonzentration bis zur Tropfnässe besprüht 1, bzw. 7 Tage nach der Applikation wurden die Unterseiten der Blätter mit einer wässrigen Sporangienaufschwemmung von *Plasmopara viticola* inokuliert. Danach wurden die Reben zunächst für 48 Stunden in einer wasserdampfgesättigten Kammer bei 24°C und anschließend für 5 Tage im Gewächshaus bei Temperaturen zwischen 20 und 30°C aufgestellt. Nach dieser Zeit wurden die Pflanzen zur Beschleunigung des Sporangienträgerausbruchs abermals für 16 Stunden in eine feuchte Kammer gestellt. Dann wurde das Ausmaß der Befallsentwicklung auf den Blattunterseiten visuell ermittelt.

In den Tests mit 1 Tag protektiver Behandlung zeigten die mit 63 ppm der Verbindungen I-1, 1-4, bzw. I-5 behandelten Pflanzen maximal 3% Befall, während die unbehandelten Pflanzen zu 90% befallen waren.

In einer anderen Versuchsanstellung mit 7 Tagen protektiver Behandlung zeigten die mit 250 ppm der Verbindungen I-1, bzw. I-2 behandelten Pflanzen maximal 5 % Befall, während die unbehandelten Pflanzen zu 70 % befallen waren.

## Patentansprüche

1. 5-Alkoxyalkyl-6-alkyl-7-amino-azolopyrimidine der Formel I 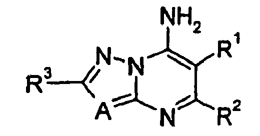 in der die Substituenten folgende Bedeutung haben:
R¹ C₁-C₁₂-Alkyl, C₃-C₆-Cycloalkyl, C₂-C₁₂-Alkenyl, C₂-C₁₂-Alkinyl, C₂-C₁₂-Alkoxyalkyl, C₂-C₁₂-Cyanoalkyl und C₈-C₁₉-Benzyloxyalkyl, wobei die Gruppen im aliphatischen oder aromatischen Teil unsubstituiert oder durch eine bis drei Gruppen R^{a} substituiert sein können:
R^{a} Halogen, Cyano, Nitro, Hydroxy, C₃-C₆-Cycloalkyl, C₁-C₆-Alkoxy, C₁-C₆-Alkylthio und NR^{A}R^{B};
R^{A}, R^{B} Wasserstoff und C₁-C₆-Alkyl;
R² C₁-C₁₂-Alkoxy-C₁-C₁₂-alkyl, Phenoxy-C₁-C₁₂-alkyl, C₁-C₁₂-Alkylthio-C₁-C₁₂-alkyl und Phenylthio-C₁-C₁₂-alkyl, in welchen Gruppen die Kohlenstoffketten durch eine bis drei Gruppen R^{a} substituiert und die Phenylringe durch einen bis fünf Substituenten aus C₁-C₆-Alkyl oder einer Gruppe R^{a} substituiert sein können;
R³ Wasserstoff und C₁-C₆-Alkyl;
A N und C-R^{A}.

2. Verbindungen der Formel I gemäß Anspruch 1, in der
R² C₁-C₁₂-Alkoxy-C₁-C₁₂-alkyl und C₁-C₁₂-Alkylthio-C₁-C₁₂-alkyl, in welchen Gruppen die Kohlenstoffketten unsubstituiert oder durch eine bis drei Gruppen R^{a} substituiert sein können.

3. Verbindungen der Formel I gemäß Anspruch 1 oder 2, in der die Substituenten folgende Bedeutung haben:
R¹ C₁-C₁₂-Alkyl, C₃-C₈-Cycloalkyl, C₂-C₁₂-Alkenyl, C₂-C₁₂-Alkinyl, C₂-C₁₂-Alkoxyalkyl, C₂-C₁₂-Cyanoalkyl, C₁-C₁₂-Halogenalkyl, C₁-C₁₂-Hydroxyalkyl und C₈-C₁₉-Benzyloxyalkyl; und
R² C₁-C₁₂-Alkoxy-C₁-C₁₂-alkyl.

4. Verbindungen der Formel I gemäß einem der Ansprüche 1 bis 3, worin R¹ für eine unsubstituierte unverzweigte oder ein-, zwei- oder dreifach verzweigte C₁-C₁₂-Alkyl-, C₂-C₁₂-Cyanoalkyl-, C₁-C₁₂-Halogenalkyl- oder C₁-C₁₂-Hydroxyalkylkette steht.

5. Verbindungen der Formel I gemäß einem der Ansprüche 1 bis 4, worin R² für C₁-C₁₂-Alkoxymethyl steht.

6. Verbindungen der Formel I gemäß Anspruch 1, welche der Formel I.A 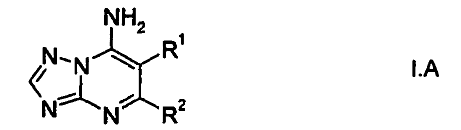 entsprechen, in der die Variablen die Bedeutung gemäß einem der Ansprüche 1 bis 4 aufweisen.

7. Verfahren zur Herstellung von Verbindungen der Formel I gemäß einem der Ansprüche 1 bis 5, **dadurch gekennzeichnet, dass** man β-Ketoester der Formel II, 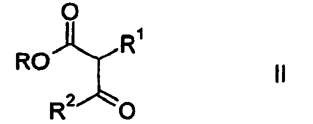 in der R für C₁-C₄-Alkyl steht, mit 3-Amino-1,2,4-triazol oder-pyrazol der Formel III 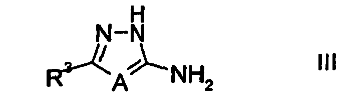 zu 7-Hydroxyazolopyrimidinen der Formel IV 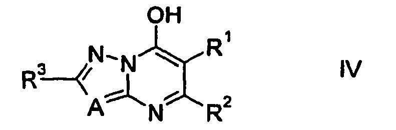 umsetzt, welche zu Verbindungen der Formel V, 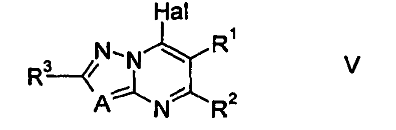 in der Hal für Chlor oder Brom steht, halogeniert werden, und V mit Ammoniak umgesetzt wird.

8. Verbindungen der Formeln IV und V gemäß Anspruch 7, worin R¹ für eine unverzweigte oder ein-, zwei-, drei- oder mehrfach verzweigte C₅-C₁₂-Alkylgruppe oder C₅-C₁₀-Alkoxypropylgruppe steht.

9. Verfahren zur Herstellung von Verbindungen der Formel I gemäß Anspruch 1 oder 2, **dadurch gekennzeichnet, dass** man Acylcyanide der Formel VI, 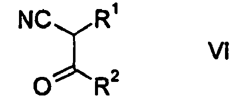 mit 3-Amino-1,2,4-triazol oder-pyrazol der Formel III gemäß Anspruch 7 umsetzt.

10. Mittel, enthaltend einen festen oder flüssigen Träger und eine Verbindung der Formel I gemäß Anspruch 1 oder 2.

11. Mittel gemäß Anspruch 10, enthaltend einen weiteren Wirkstoff.

12. Saatgut, enthaltend eine Verbindung der Formel I gemäß Anspruch 1 oder 2 in einer Menge von 1 bis 1000 g pro 100 kg.

13. Verfahren zur Bekämpfung von pflanzenpathogenen Schadpilzen, **dadurch gekennzeichnet, dass** man die Pilze, oder die vor Pilzbefall zu schützenden Materialien, Pflanzen, den Boden oder Saatgüter mit einer wirksamen Menge einer Verbindung der Formel I gemäß Anspruch 1 oder 2 behandelt.

## Claims

1. A 5-alkoxyalkyl-6-alkyl-7-aminoazolopyrimidine of the formula I 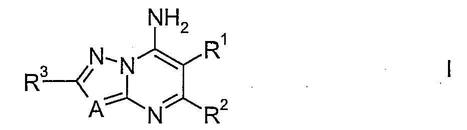 in which the substituents are defined as follows:
R¹ is C₁-C₁₂-alkyl, C₃-C₆-cycloalkyl, C₂-C₁₂-alkenyl, C₂-C₁₂-alkynyl, C₂-C₁₂-alkoxyalkyl, C₂-C₁₂-cyanoalkyl, and C₈-C₁₉-benzyloxyalkyl, where the groups in the aliphatic or aromatic moiety may be unsubstituted or may have substitution by from one to three groups R^{a}:
R^{a} is halogen, cyano, nitro, hydroxy, C₃-C₆-cycloalkyl, C₁-C₆-alkoxy, C₁-C₆-alkylthio, and NR^{A}R^{B} ;
R^{A}, R^{B} are hydrogen and C₁-C₆-alkyl ;
R² is C₁-C₁₂-alkoxy-C₁-C₁₂-alkyl, phenoxy-C₁-C₁₂-alkyl, C₁-C₁₂-alkylthio-C₁-C₁₂-alkyl, and phenylthio-C₁-C₁₂-alkyl, in which groups the carbon chains can have substitution by from one to three groups R^{a}, and the phenyl rings can have substitution by from one to five substituents composed of C₁-C₆-alkyl or of a group R^{a};
R³ is hydrogen and C₁-C₆-alkyl;
A is N and C-R^{A}.

2. The compound of the formula I according to claim 1, in which
R² is C₁-C₁₂-alkoxy-C₁-C₁₂-alkyl and C₁-C₁₂-alkylthio-C₁-C₁₂-alkyl, in which groups the carbon chains may be unsubstituted or may have substitution by from one to three groups R^{a}.

3. The compound of the formula I according to claim 1 or 2, in which the substituents are defined as follows:
R¹ is C₁-C₁₂-alkyl, C₃-C₆-cycloalkyl, C₂-C₁₂-alkenyl, C₂-C₁₂-alkynyl, C₂-C₁₂-alkoxyalkyl, C₂-C₁₂-cyanoalkyl, C₁-C₁₂-haloalkyl, C₁-C₁₂-hydroxyalkyl, and C₈-C₁₉-benzyloxyalkyl; and
R² is C₁-C₁₂-alkoxy-C₁-C₁₂-alkyl.

4. The compound of the formula I according to any of claims 1 to 3, where R¹ is an unsubstituted unbranched or singly, doubly, or triply branched C₁-C₁₂-alkyl chain, C₂-C₁₂-cyanoalkyl chain, C₁-C₁₂-haloalkyl chain, or C₁-C₁₂-hydroxyalkyl chain.

5. The compound of the formula I according to any of claims 1 to 4, where R² is C₁-C₁₂-alkoxymethyl.

6. The compound of the formula I according to claim 1, which corresponds to the formula I.A 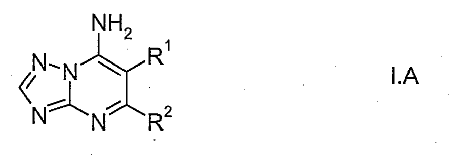 in which the variables are defined according to any of claims 1 to 4.

7. A process for preparation of compounds of the formula I according to any of claims 1 to 5, which comprises reacting β-ketoesters of the formula II, 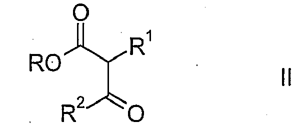 in which R is C₁-C₄-alkyl, with 3-amino-1,2,4-triazole or -pyrazole of the formula III 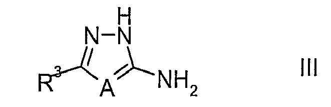 to give 7-hydroxyazolopyrimidines of the formula IV 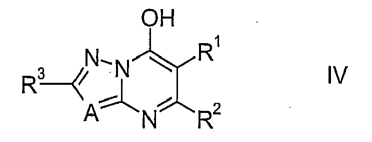 which are halogenated to give compounds of the formula V, 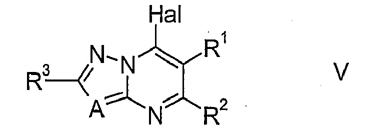 where Hal is chlorine or bromine, and reacting V with ammonia.

8. The compound of the formula IV and the compound of the formula V according to claim 7.

9. A process for preparation of compounds of the formula I according to claim 1 or 2, which comprises reacting acylcyanides of the formula VI, 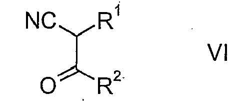 with 3-amino-1,2,4-triazole or -pyrazole of the formula III according to claim 7.

10. A composition comprising a solid or liquid carrier and a compound of the formula I according to claim 1 or 2.

11. The composition according to claim 10, comprising a further active ingredient.

12. A seed material comprising, per 100 kg, an amount of from 1 to 1000 g of a compound of the formula I according to claim 1 or 2.

13. A method for controlling phytopathogenic harmful fungi, which comprises treating the fungi, or the materials to be protected from fungal infestation, or plants, or the soil, or seed materials, with an effective amount of a compound of the formula I according to claim 1 or 2.

## Revendications

1. 5-alcoxyalkyl-6-alkyl-7-amino-azolopyrimidines selon la formule I : 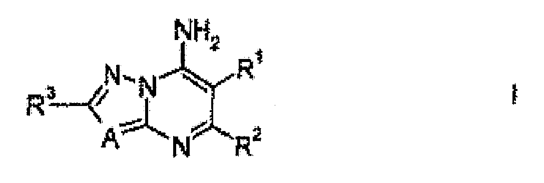 dans laquelle les substituants ont la signification suivante :
R¹ représente un alkyle en C₁-C₁₂, un cycloalkyle en C₃-C₆, un alcényle en C₂-C₁₂, un alcynyle en C₂-C₁₂, un alcoxyalkyle en C₂-C₁₂, un cyanoalkyle en C₂-C₁₂ et un benzyloxyalkyle en C₈-C₁₉, les groupes pouvant être non substitués dans la partie aliphatique ou aromatique ou pouvant être substitués par un à trois groupes R^{a} :
R^{a} représente un radical halogène, cyano, nitro, hydroxy, un cycloalkyle en C₃-C₆, un alcoxy en C₁-C₆, un alkylthio en C₁-C₆ et un NR^{A}R^{B} ;
R^{A} et R^{B} représentent de l'hydrogène et un alkyle en C₁-C₆ ;
R² représente un alcoxy en C₁-C₁₂-alkyle en C₁-C₁₂, un phénoxy-alkyle en C₁-C₁₂, un alkylthio en C₁-C₁₂-alkyle en C₁-C₁₂ et un phénylthio-alkyle en C₁-C₁₂, groupes dans lesquels les chaînes de carbone peuvent être substituées par un à trois groupes R^{a} et les cycles de phényle peuvent être substitués par un à cinq substituants à partir d'alkyle en C₁-C₆ ou d'un groupe R^{a} ;
R³ représente de l'hydrogène et un alkyle en C₁-C₆ ;
A représente N et C-R^{A}.

2. Composés selon la formule I suivant la revendication 1 dans laquelle :
R² représente un alcoxy en C₁-C₁₂-alkyle en C₁-C₁₂ et un alkylthio en C₁-C₁₂-alkyle en C₁-C₁₂, groupes dans lesquels les chaînes de carbone peuvent être non substituées ou peuvent être substituées par un à trois groupes R^{a}.

3. Composés selon la formule I suivant la revendication 1 ou 2, dans laquelle les substituants ont la signification suivante :
R¹ représente un alkyle en C₁-C₁₂, un cycloalkyle en C₃-C₆, un alcényle en C₂-C₁₂, un alcynyle en C₂-C₁₂, un alcoxyalkyle en C₂-C₁₂, un cyanoalkyle en C₂-C₁₂, un halogénoalkyle en C₁-C₁₂, un hydroxyalkyle en C₁-C₁₂ et un benzyloxyalkyle en C₈-C₁₉ ; et
R² représente un alcoxy en C₁-C₁₂-alkyle en C₁-C₁₂.

4. Composés selon la formule I suivant l'une des revendications 1 à 3, dans laquelle R¹ représente une chaîne alkyle en C₁-C₁₂, cyanoalkyle en C₂-C₁₂, halogénoalkyle en C₁-C₁₂ ou hydroxyalkyle en C₁-C₁₂ non substituée, non ramifiée ou ramifiée à une, deux ou trois reprises.

5. Composés selon la formule I suivant l'une des revendications 1 à 4 dans laquelle R² représente un alcoxyméthyle en C₁-C₁₂.

6. Composés selon la formule I suivant la revendication 1, qui correspondent à la formule I.A : 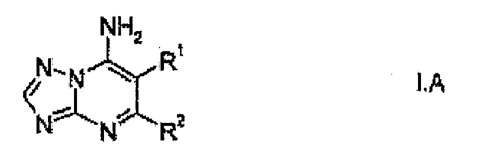 dans laquelle les variables présentent la signification selon l'une des revendications 1 à 4.

7. Procédé de préparation des composés selon la formule I suivant l'une des revendications 1 à 5, **caractérisé en ce que** l'on fait réagir des esters β-céto selon la formule II : 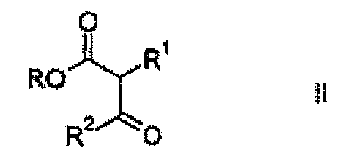 dans laquelle R représente un alkyle en C₁-C₄, avec un 3-amino-1,2,4-triazol ou un 3-amino-1,2,4-pyrazol selon la formule III : 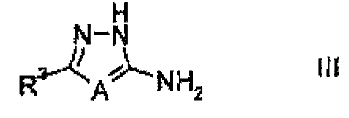 pour donner des 7-hydroxyazolopyrimidines selon la formule IV : 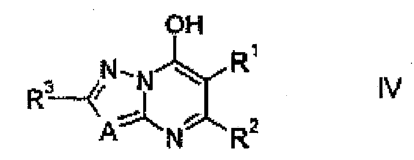 qui sont halogénées en composés selon la formule V : 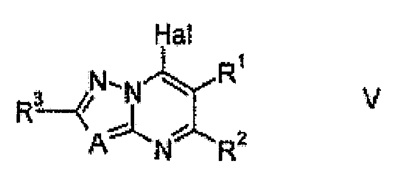 dans laquelle Hal représente du chlore ou du brome, et **en ce qu'**on fait réagir V avec de l'ammoniac.

8. Composés selon les formules IV et V suivant la revendication 7, dans lesquelles R¹ représente un groupe alkyle en C₅-C₁₂ non ramifié ou ramifié à une, deux, trois ou plusieurs reprises, ou un groupe alcoxypropyle en C₅-C₁₀.

9. Procédé de préparation des composés selon la formule I suivant la revendication 1 ou 2, **caractérisé en ce que** l'on fait réagir des acylcyanures selon la formule VI : 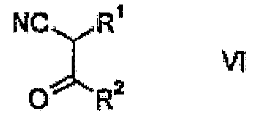 avec un 3-amino-1,2,4-triazol ou un 3-amino-1,2,4-pyrazol selon la formule III suivant la revendication 7.

10. Agent contenant un support solide ou liquide et un composé selon la formule I suivant la revendication 1 ou 2.

11. Agent suivant la revendication 10, contenant une autre matière active.

12. Semence contenant un composé selon la formule I suivant la revendication 1 ou 2 dans une quantité de 1 à 1000 g par 100 kg.

13. Procédé de lutte contre les champignons nuisibles phytopathogènes, **caractérisé en ce que** l'on traite les champignons ou les matériaux, plantes, sols ou semences à protéger d'une infestation par les champignons avec une quantité efficace d'un composé selon la formule I suivant la revendication 1 ou 2.
